(19) Europäisches Patentamt
European Patent Office
Office européen des brevets

(11) EP 2 891 695 A1

(12) **EUROPEAN PATENT APPLICATION**
published in accordance with Art. 153(4) EPC

(43) Date of publication:
**08.07.2015 Bulletin 2015/28**

(21) Application number: **13832696.2**

(22) Date of filing: **05.08.2013**

(51) Int Cl.:
***C09K 11/06** (2006.01)* ***H01L 51/50** (2006.01)*
***C07C 211/56** (2006.01)*

(86) International application number:
**PCT/JP2013/071132**

(87) International publication number:
**WO 2014/034384 (06.03.2014 Gazette 2014/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **31.08.2012 JP 2012190965**

(71) Applicants:
- **Kyushu University**
**Higashi-ku**
**Fukuoka-shi, Fukuoka 812-8581 (JP)**
- **LINTEC Corporation**
**Tokyo 173-0001 (JP)**

(72) Inventors:
- **TANEDA Masatsugu**
**Kashiwara-shi, Osaka 5828582 (JP)**
- **ADACHI Chihaya**
**Fukuoka-shi**
**Fukuoka 812-8581 (JP)**
- **NAKATA Yasukazu**
**Tokyo 173-0001 (JP)**

(74) Representative: **Müller-Boré & Partner Patentanwälte PartG mbB**
**Friedenheimer Brücke 21**
**80639 München (DE)**

# (54) ORGANIC LUMINESCENT MATERIAL, METHOD FOR PROUDCING ORGANIC LUMINESCENT MATERIAL AND ORGANIC LUMINESCENT ELEMENT

(57) Provided are an organic luminescent material exhibiting excellent horizontal orientation or the like when produced into a film, an efficient method for producing such an organic luminescent material, and an organic light emitting element using such an organic luminescent material. This organic luminescent material or the like is used as a host material and is represented by the following general formula (1), having a donor-acceptor-type molecular structure containing an electron acceptor-like tetrafluoroarylene structure in its central part and a diphenylamine structure linked to each of the two ends of the tetrafluoroarylene structure through an electron donor-like arylene group;

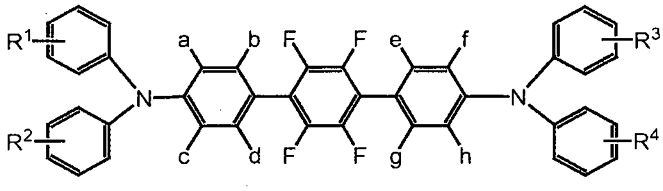

(1)

in the general formula (1), the substituents $R^1$ to $R^4$ and a to h each independently represent a hydrogen atom, an alkyl group having 1 to 20 carbon atoms, a substituted alkyl group having 1 to 20 carbon atoms, an aryl group having 6 to 20 carbon atoms, a substituted aryl group having 6 to 20 carbon atoms, or an amino group.

EP 2 891 695 A1

Fig.1

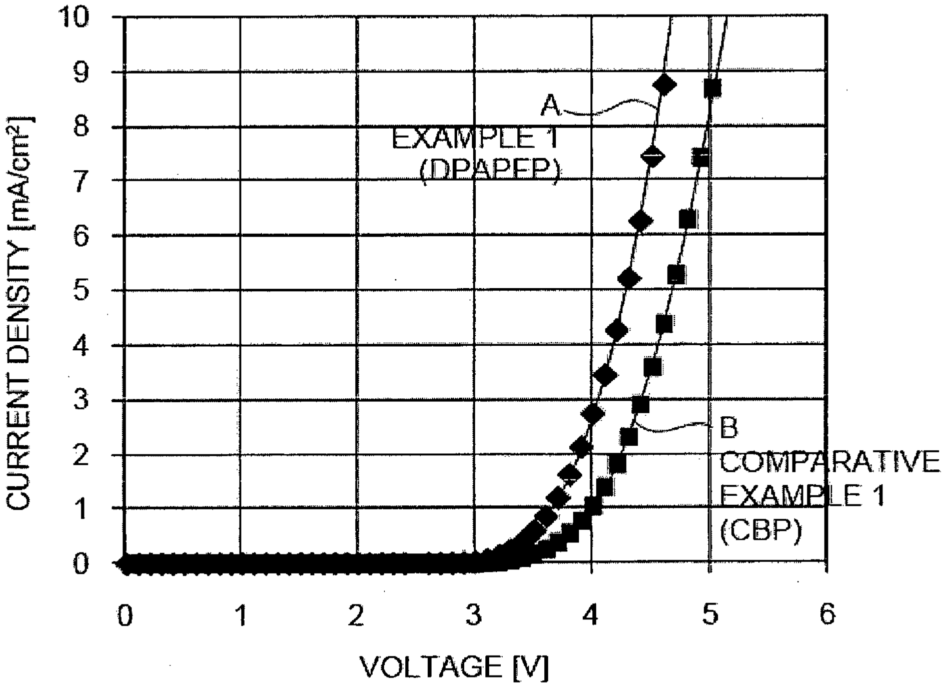
10
9
8
7
6
5
4
3
2
1
0
0
1
2
3
4
5
6
CURRENT DENSITY [mA/cm²]
VOLTAGE [V]
A
EXAMPLE 1
(DPAPFP)
B
COMPARATIVE
EXAMPLE 1
(CBP)

## Description

### TECHNICAL FIELD

The present invention relates to an organic luminescent material, a method for producing an organic luminescent material, and an organic luminescent element. In particular, the present invention relates to an organic luminescent material exhibiting excellent horizontal orientation and the like when formed into a film (hereinafter, may be referred to as an orientational luminescent material), a method for producing such an organic luminescent material, and an organic light emitting element formed by using such an organic luminescent material as a host material.

### BACKGROUND ART

It has been hitherto suggested, as an attempt to enhance the luminescence efficiency of organic electroluminescent elements (organic EL elements), to use phosphorescence instead of fluorescence.

That is, it is expected to achieve high luminescence efficiency when, in the light emitting layer of an organic EL element, a phosphorescent light emitting material is included in a predetermined amount with respect to a host material as a main component, and when, at the same time, the excited triplet state of the phosphorescent light emitting material is principally used. It is because it may be considered that, when electrons and holes recombine in the organic EL element, an excited singlet state and an excited triplet state having different spin multiplicities are produced at a ratio of 1:3.

Therefore, in the case of fluorescence, which is the light emitted at the time of returning from the excited singlet state to the ground state, only about 25% of excitons (100%) can be used, while in the case of phosphorescence, which is the light emitted at the time of returning from the excited triplet state to the ground state, many excitons can be used. That is, since excitons may be converted to excitons that emit phosphorescent light by intersystem crossing, an excited singlet state is converted to an excited triplet state and it may be possible to use of 100% of the excitons. Thus, improvement in the luminescence efficiency is expected.

Thus, in order to increase the luminescence efficiency, there has been suggested an organic EL element including a light emitting layer formed by using a carbazole compound as a host material and doping the host material with a phosphorescent iridium complex material (see, for example, Patent Document 1).

More specifically, an organic EL element is formed by sequentially laminating a positive electrode, a light emitting layer containing a phosphorescent iridium complex material, an electron transport layer containing an organic compound, and a negative electrode, in which the light emitting layer has a carbazole compound as a host material and contains an iridium complex material in an amount of 0.5% to 8% by weight.

In addition, as a representative phosphorescent iridium complex material, tris(2-phenylpyridine)iridium (hereinafter, may be referred to as $Ir(PPY)_3$) represented by the following formula (A) has been disclosed.

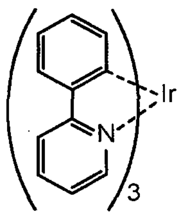 (A)

Furthermore, in order to improve the luminescence characteristics and the lifetime of the element, there have been proposed a phosphorescent organic metal complex having a predetermined structure, and a light emitting element containing, in its light emitting layer, the phosphorescent organic metal complex (see, for example, Patent Document 2).

More specifically, there has been proposed a light emitting element (organic EL element) containing, in a emitting layer, a phosphorescent organic metal complex in which β-dicarbonyls located at one end of a long carbon chain, represented by the following formula (B), and two molecules of 2-phenylpyridine are coordinated to a platinum atom or the like, and in which β-dicarbonyls located at the other end of the carbon chain have adopted the same coordinated structure.

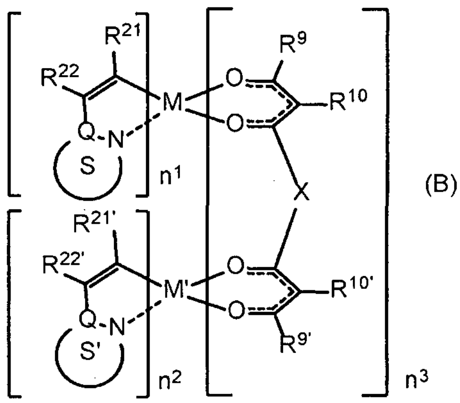

(B)

On the other hand, there has been suggested an organic light emitting element, which exhibits high efficiency and a long lifetime, formed by using a predetermined fluorine-containing triphenylamine compound as a dopant material and by using a predetermined host material (see, for example, Patent Literature 3).

More specifically, the organic light emitting element uses, in its light emitting layer, a fluorine-containing triphenylamine compound, represented by the following formula (C), as a dopant material and a fluorene compound, represented by the following formula (D) or (E), as a host material.

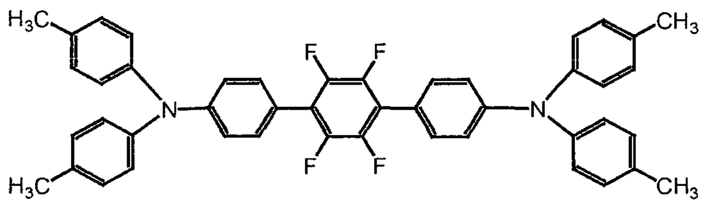

(C)

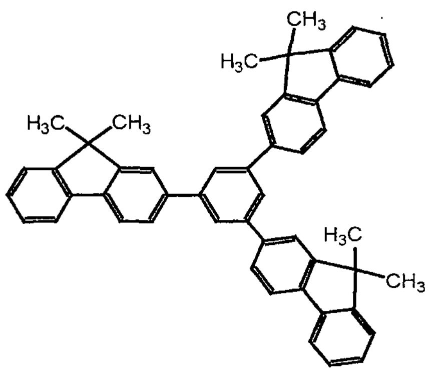

(D)

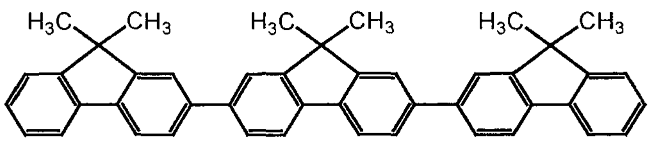

(E)

Patent Document 1: JP 2001-313178 A (claims and the like)
Patent Document 2: JP 2007-277170 A (claims and the like)
Patent Document 3: Japanese Patent No. 4311707 (claims and the like)

DISCLOSURE OF THE INVENTION

PROBLEMS TO BE SOLVED BY THE INVENTION

Here, the phosphorescent iridium complex material disclosed in Patent Document 1 as well as the phosphorescent organic metal complex disclosed in Patent Document 2 exhibit insufficient horizontal orientation when respectively produced into films, and transition moments are not aligned. Therefore, arises the problem that a high luminance phosphorescent light emitting element may still not be obtained.

Furthermore, the phosphorescent iridium complex material disclosed in Patent Document 1 as well as the phosphorescent organic metal complex disclosed in Patent Document 2 also have the problem that, when used respectively in the light emitting layers of organic EL elements, the range of the amount of additives that can be incorporated with respect to the carbazole compound and the like, used as the host material, is narrow.

Furthermore, the problem of the fluorine-containing triphenylamine compound disclosed in Patent Document 3 is that the luminescence efficiency is still low, because the compound is used as a dopant material and is not capable of emitting phosphorescent light.

In addition, the organic luminescent material disclosed in Patent Document 3 must use a predetermined fluorene compound as the host material. The problem is that the cost of the organic luminescent material thus obtainable is high, and this is economically disadvantageous.

In addition, as in the case of the organic luminescent material disclosed in Patent Document 3, when a methyl group is introduced at the para-position of the terminal phenyl group, the amorphousness of the thin film formed from organic molecules tends to be significantly decreased. Therefore, it has been speculated that, even though the organic luminescent material is suitable for the use as a dopant material, it is conventionally disadvantageous to use it as a host material.

Thus, under circumstances such as described above, the inventors of the present invention have found that, by introducing a predetermined donor-acceptor structure into the molecule, the relevant compound exhibits satisfactory horizontal orientation or the like when produced into a film. Also, when used as a host material of an organic luminescent material, even if a low voltage is applied, along with a relatively high current value, high external luminescence efficiency (EQE) is obtained. Thus, the inventors completed the present invention.

That is, an object of the present invention is to provide an organic luminescent material exhibiting satisfactory horizontal orientation or the like, an efficient method for producing such an organic luminescent material, and an organic light emitting element that is formed by using such an organic luminescent material and can be driven at a low voltage or the like.

MEANS FOR SOLVING THE PROBLEMS

According to the present invention, there is provided an organic luminescent material represented by the following general formula (1), which has a donor-acceptor-type molecular structure containing an electron acceptor-like tetrafluoroarylene structure in its central part and a diphenylamine structure linked to each of the two ends of the tetrafluoroarylene structure through an electron donor-like arylene group, and which is used as a host material. Thus, the problems described above can be solved.

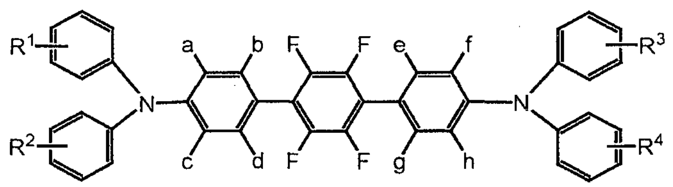
(1)

In the general formula (1), the substituents $R^1$ to $R^4$ and a to h each independently represent a hydrogen atom, an alkyl group having 1 to 20 carbon atoms, a substituted alkyl group having 1 to 20 carbon atoms, an aryl group having 6 to 20 carbon atoms, a substituted aryl group having 6 to 20 carbon atoms, or an amino group.

That is, since the molecules of the compound contain a donor-acceptor-type molecular structure, when a film is produced therefrom, excellent horizontal orientation and the like may be obtained.

Therefore, when such an organic luminescent material is used as a host material for the light emitting layer of an organic EL element (phosphorescent light emitting element), even if a low voltage is applied, a relatively high current value is obtained, and high external quantum efficiency (EQE) may be obtained by applying a small electric current.

Furthermore, it is speculated that the diphenylamine structure present at both ends respectively enhance amorphousness, and with such an organic luminescent material, overall crystallization of the host material may be

effectively suppressed. Also, even if a relatively extensive amount of the dopant material is incorporated, the dopant material may be mixed and dispersed uniformly.

Meanwhile, a donor-acceptor-type molecular structure is basically configured such that an electron donor-like arylene group, an electron acceptor-like tetrafluoroarylene structure, and an electron donor-like arylene group are arranged in this order; however, the donor-acceptor-type molecular structure may be considered as one electron donor-like structure containing a diphenylamine structure linked to the ends of electron donor-like arylene groups.

That is, it is contemplated that the structure is configured such that an arylene group containing an electron donor-like diphenylamine structure, an electron acceptor-like tetrafluoroarylene structure, and an arylene group containing an electron donor-like diphenylamine structure are arranged in this order.

Furthermore, when configuring the organic luminescent material of the present invention, it is preferable that the substituents $R^1$ to $R^4$ and a to h each independently represent a hydrogen atom or an alkyl group having 1 to 4 carbon atoms.

When such a configuration is adopted, an organic luminescent material, which can be produced relatively easily, is inexpensive, and has stable properties, may be obtained.

Furthermore, when configuring the organic luminescent material of the present invention, it is preferable for the order parameter calculated from the anisotropy of the extinction coefficient to have a value within the range of -0.5 to -0.1.

When the organic luminescent material is configured by defining the order parameter as such, horizontality of the organic luminescent material may be quantitatively controlled. Furthermore, when a predetermined organic light emitting element is configured, even if a small electric current is applied, low voltage driving may be enabled, and the service life or efficiency may be improved.

Furthermore, when configuring the organic luminescent material of the present invention, in a three-dimensional space formed by the XYZ-axes, when the angle formed by the Z-axis, which is a vertical axis, and the virtual axis line direction of the molecule of the organic luminescent material is designated as $\theta$, it is preferable for the horizontal angle ($\theta 2$) represented by ($90° - \theta$) to be adjusted to a value of 31° or less.

When the organic luminescent material is configured by defining the horizontal angle as such, horizontality of the organic luminescent material may be quantitatively managed, and when a predetermined organic light emitting element is configured, even if a low voltage is applied, a relatively high current value may be obtained. Thus, the lifetime or efficiency may be improved.

Furthermore, according to another aspect of the present invention, there is provided a method for producing an organic luminescent material represented by the following general formula (1), which has a donor-acceptor-type molecular structure containing an electron acceptor-like tetrafluoroarylene structure in its central part and a diphenylamine structure linked to each of the two ends of the tetrafluoroarylene structure through an electron donor-like arylene group, and which is used as a host material.

Further, the method is a method for producing an organic luminescent material, the method including a first step of preparing a halogenated aryl from 1,4-dihalogenated tetrafluoroarylene; a second step of respectively preparing a first boronic acid ester formed from para-aminoarylboronic acid ester having the substituents $R^1$ and $R^2$, and a second boronic acid ester formed from para-aminoarylboronic acid ester having the substituents $R^3$ and $R^4$; and a third step of cross-coupling the halogen atom at one end of the halogenated aryl and the first boronic acid ester under the action of a palladium catalyst and a basic nucleophile, and then cross-coupling the halogen atom at the other end of the halogenated aryl and the second boronic acid ester under the action of a palladium catalyst and a basic nucleophile;

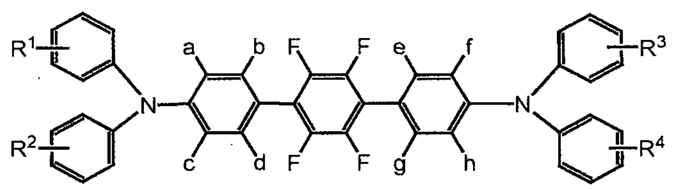 (1)

in the general formula (1), the substituents $R^1$ to $R^4$ and a to h each independently represent a hydrogen atom, an alkyl group having 1 to 20 carbon atoms, a substituted alkyl group having 1 to 20 carbon atoms, an aryl group having 6 to 20 carbon atoms, a substituted aryl group having 6 to 20 carbon atoms, or an amino group.

As the method is carried out as such, a predetermined organic luminescent material exhibiting excellent horizontal orientation or the like, if formed into a film, may be efficiently produced.

Therefore, when such an organic luminescent material is used as a host material of the light emitting layer in an organic EL element, even if a low voltage is applied, a relatively high current value may be obtained. Also, high external quantum efficiency (EQE) may be obtained by applying a small electric current.

In addition, in the case for which the substituents $R^1$ and $R^2$ and the substituents $R^3$ and $R^4$ are of different

types, that is, even if the organic luminescent material is an organic luminescent material having an asymmetric structure, or in a case in which the substituents $R^1$ and $R^2$ and the the substituents $R^3$ and $R^4$ are of the same type, that is, even if the organic luminescent material is an organic luminescent material having a symmetric structure, the materials may be respectively produced efficiently by, for example, a two-stage process.

Furthermore, when carrying out the method for producing the organic luminescent material of the present invention, it is preferable that, in the third step, cross-coupling is carried out by, respectively, adding the first boronic acid ester and the second boronic acid ester dropwise to the halogenated aryl.

When the method is carried out as such, even if the lifetime of activity of the boronic acid esters is short, fresh boronic acid esters are supplied all the time through dropwise addition. Therefore, the boronic acid esters may be used effectively in cross-coupling, and a predetermined organic luminescent material may be efficiently produced, regardless of whether the organic luminescent material is of a symmetric type or an asymmetric type.

That is, when the method is carried out as such, even a halogenated aryl, that is considered to have strong electron acceptor-like properties and very low reactivity in Suzuki coupling or the like, may react effectively with the predetermined boronic acid esters. Thus, the yield of the predetermined organic luminescent material may be dramatically enhanced.

Furthermore, according to yet another aspect of the present invention, there is provided a method for producing an organic luminescent material represented by the following general formula (1'), which has a donor-acceptor-type molecular structure containing an electron acceptor-like tetrafluoroarylene structure in its central part and a diphenylamine structure linked to each of the two ends of the tetrafluoroarylene structure through an electron donor-like arylene group, and which is used as a host material.

Also, the method is a method for producing an organic luminescent material, the method including a first step of preparing a halogenated aryl formed from 1,4-dihalogenated tetrafluoroarylene; a second step of preparing a boronic acid ester formed from a para-aminoarylboronic acid ester having the substituents $R^1$ and $R^2$ or the substituents $R^3$ and $R^4$; and a third step of cross-coupling the halogenated aryl and the boronic acid ester under the action of a palladium catalyst and a basic nucleophile;

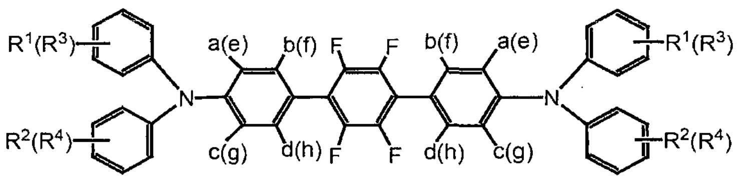

(1')

in the general formula (1'), the substituents $R^1$ and $R^2$ (or $R^3$ and $R^4$) and a to d (or e to h) each independently represent a hydrogen atom, an alkyl group having 1 to 20 carbon atoms, a substituted alkyl group having 1 to 20 carbon atoms, an aryl group having 6 to 20 carbon atoms, a substituted aryl group having 6 to 20 carbon atoms, or an amino group.

When the method is carried out as such, even in case the lifetime of activity of the boronic acid ester is short, a fresh boronic acid ester is supplied all the time through dropwise addition. Therefore, a symmetric organic luminescent material may be produced efficiently by effectively using the boronic acid ester in cross-coupling.

That is, even a halogenated aryl that is considered to have strong electron acceptor-like properties and very low reactivity in Suzuki coupling or the like, may react effectively with a predetermined boronic acid ester. Thus, the yield of the predetermined organic luminescent material can be dramatically enhanced.

Furthermore, according to yet another aspect of the present invention, there is provided an organic light emitting element including a light emitting layer which uses, as a host material, an organic luminescent material represented by the following general formula (1), the organic luminescent material having a donor-acceptor-type molecular structure containing an electron acceptor-like tetrafluoroarylene structure in its central part and a diphenylamine structure linked to each of the two ends of the tetrafluoroarylene structure through an electron donor-like arylene group, and which is formed by incorporating a dopant material thereto;

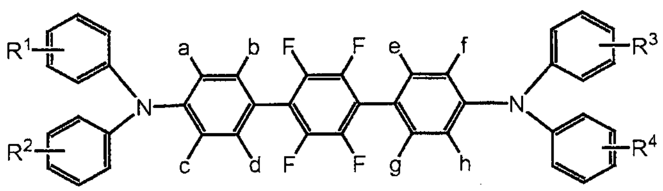

(1)

in the general formula (1), the substituents $R^1$ to $R^4$ and a to h each independently represent a hydrogen atom, an alkyl group having 1 to 20 carbon atoms, a substituted alkyl group having 1 to 20 carbon atoms, an aryl group having

6 to 20 carbon atoms, a substituted aryl group having 6 to 20 carbon atoms, or an amino group.

When such a configuration is adopted, even if a low voltage is applied, a relatively high current value is obtained. Also, high external quantum efficiency (EQE) may be obtained by applying a small electric current.

Furthermore, when configuring the organic light emitting element of the present invention, it is preferable that the dopant material is composed of an iridium complex compound and a platinum complex compound, or any one of them.

When such a configuration is adopted, high luminance light emission (phosphorescence) may be obtained with more stability and for a long time, by applying a relatively small electric current.

Furthermore, when the organic light emitting element of the present invention, it is preferable that the dopant material is represented by the following general formula (2), and is a horizontally orientational compound having a straight-chained conjugated structure and having a 2-phenylpyridine ligand, a coordinating metal, and an acetylacetonate ligand in the molecule;

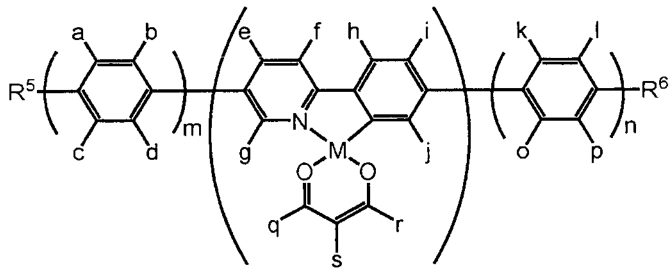

(2)

in the general formula (2), $R^5$ and $R^6$ each independently represent an alkyl group having 1 to 20 carbon atoms, a substituted alkyl group having 1 to 20 carbon atoms, an aryl group having 6 to 20 carbon atoms, a substituted aryl group having 6 to 20 carbon atoms, or an amino group; a to 1 and o to s each independently represent a hydrogen atom, an alkyl group having 1 to 20 carbon atoms, a substituted alkyl group having 1 to 20 carbon atoms, an aryl group having 6 to 20 carbon atoms, a substituted aryl group having 6 to 20 carbon atoms, or a halogen atom; coordinating metal M represents platinum (Pt), iridium (Ir), nickel (Ni), copper (Cu), or gold (Au); and the numbers of repetition, m and n each independently represent an integer from 0 to 4, while m + n is an integer of 1 or greater.

When such a configuration is adopted, horizontal orientation may be further enhanced by the action of the dopant material, and high luminance light emission (phosphorescence) may be obtained in a stable way and for a long time by applying a relatively small electric current.

Moreover, since a predetermined dopant material exhibiting superior horizontal orientation is used, when the organic luminescent material is produced into a film having a predetermined thickness and is exposed to light radiation at a predetermined angle (for example, 90° with respect to the film), phosphorescence with high polarizability in the horizontal direction with respect to the substrate may be obtained. Also, with respect to the host material as the main component, an extensive amount of dopant material can be incorporated.

## BRIEF DESCRIPTION OF DRAWINGS

Fig. 1 is a J-V plot of an organic EL element using an organic luminescent material exhibiting horizontal orientation (Example 1);

Fig. 2(a) is a diagram provided to explain the relationship between the external quantum efficiency of an organic EL element which uses an organic luminescent material exhibiting horizontal orientation (Example 1) and the current density, and Fig. 2(b) is a diagram provided to explain the relationship between the external quantum efficiency of an organic EL element which uses an organic luminescent material exhibiting non-horizontal orientation (Comparative Example 1);

Figs. 3(a) to 3(f) are diagrams provided to explain the effect of introducing a donor-acceptor structure in an organic luminescent material exhibiting horizontal orientation;

Figs. 4(a) to 4(b) are diagrams provided to explain the orientation state in an organic luminescent material exhibiting non-horizontal orientation (CBP);

Fig. 5 is a cross-sectional diagram of a fundamental organic EL element;

Fig. 6 is a cross-sectional diagram of a modification example of an organic EL element including an electron injection layer;

Fig. 7 is a diagram illustrating the relationship between the light emission time t ($\mu$sec) and the luminescence intensity in the phosphorescence emission spectrum of an organic EL element;

Fig. 8 is a diagram provided to explain the relationship between the anisotropy of the extinction coefficient (k) of an organic luminescent material exhibiting horizontal orientation (Example 1) and the wavelength (λ) ;
Fig. 9 is the NMR chart of an organic luminescent material exhibiting horizontal orientation (Example 1);
Fig. 10 is the FT-IR chart of an organic luminescent material exhibiting horizontal orientation (Example 1);
Fig. 11 is the ultraviolet absorption spectrum of an organic luminescent material exhibiting horizontal orientation (Example 1);
Fig. 12 is the light emission spectrum of an organic luminescent material exhibiting horizontal orientation (Example 1);
Fig. 13 is a schematic diagram of the molecule obtained by X-ray crystal structure analysis for explaining the molecular structure of an organic luminescent material exhibiting horizontal light emission properties (Example 1) ;
Fig. 14(a) is the NMR chart of a horizontally orientational organic luminescent material (Example 2), and Fig. 14(b) is the FT-IR chart thereof;
Fig. 15(a) is a diagram provided to explain the relationship between the anisotropy of the extinction coefficient (k) of an organic luminescent material exhibiting horizontal orientation (Example 2) and the wavelength (λ), Fig. 15(b) is the ultraviolet absorption spectrum thereof, and Fig. 15(c) is the light emission spectrum thereof;
Fig. 16(a) is the NMR chart of a horizontally orientational organic luminescent material (Example 3), and Fig. 16(b) is the FT-IR chart thereof;
Fig. 17(a) is a diagram provided to explain the relationship between the anisotropy of the extinction coefficient (k) of an organic luminescent material exhibiting horizontal orientation (Example 3) and the wavelength (λ), Fig. 17(b) is the ultraviolet absorption spectrum thereof, and Fig. 17(c) is the light emission spectrum thereof;
Fig. 18(a) is the NMR chart of a horizontally orientational organic luminescent material (Example 4), and Fig. 18(b) is the FT-IR chart thereof; and
Fig. 19(a) is a diagram provided to explain the relationship between the anisotropy of the extinction coefficient (k) of an organic luminescent material exhibiting horizontal orientation (Example 4) and the wavelength (λ), Fig. 19(b) is the ultraviolet absorption spectrum thereof, and Fig. 19(c) is the light emission spectrum.

EMBODIMENTS FOR CARRYING OUT THE INVENTION

[First embodiment]

A first embodiment of the present invention relates to an organic luminescent material represented by the following general formula (1), characterized by having a donor-acceptor-type molecular structure which contains an electron acceptor-like tetrafluoroarylene structure in its central part and a diphenylamine structure linked to each of the two ends of the tetrafluoroarylene structure through an electron donor-like arylene group, and being used as a host material.

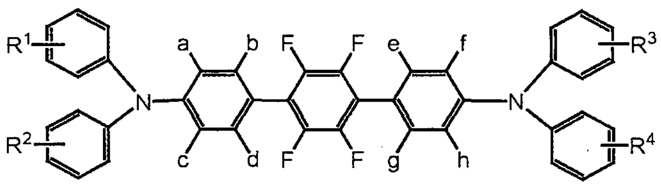

(1)

In the general formula (1), the substituents $R^1$ to $R^4$ and a to h each independently represent a hydrogen atom, an alkyl group having 1 to 20 carbon atoms, a substituted alkyl group having 1 to 20 carbon atoms, an aryl group having 6 to 20 carbon atoms, a substituted aryl group having 6 to 20 carbon atoms, or an amino group.
Hereinafter, an aspect of the organic luminescent material, which is the first embodiment of the present invention, will be described in detail with appropriate reference to the drawings.

1. Essential structure

The organic luminescent material of the first embodiment is a compound represented by the above general formula (1), characterized by containing an electron acceptor-like tetrafluoroarylene structure in its central part and containing a diphenylamine structure linked to each of the two ends of the tetrafluoroarylene structure through an electron donor-like arylene group.
That is, when an organic luminescent material as a host material has, within its molecules, a predetermined donor-acceptor-type molecular structure as an essential structure, the horizontal orientation of the molecules may be significantly enhanced when the material is produced into a film.
More specifically, the organic luminescent material of the present invention (Example 1: DPAPFP) exhibits

excellent horizontal orientation when produced into a film, and as shown by line A of the J-V characteristics of Fig. 1, a high current density is obtained even if a low voltage is applied.

For example, when a voltage of 4.0 V is applied, a current density of 3.0 mA/cm$^2$ is obtained, and in the case of 4.5 V, a current density of 7.0 mA/cm$^2$ is obtained, while in the case of 5.0 V, a current density of 15.0 mA/cm$^2$ is obtained.

On the contrary, it has been confirmed that an organic luminescent material that is said to be at maximum luminescence level at the present time (Comparative Example 1: CBP) is randomly oriented when produced into a film, and the material exhibits non-horizontal orientation.

For example, when a voltage of 4.0 V is applied, a current density of 0.8 mA/cm$^2$ is obtained, and in the case of 4.5 V, a current density of 3.0 mA/cm$^2$ is obtained, while in the case of 5.0 V, a current density of 8.0 mA/cm$^2$ is obtained.

Therefore, as shown by line B of the J-V characteristics of Fig. 1, this material exhibits inferior J-V characteristics compared to those of the present invention.

That is, it is understood that the organic luminescent material of the present invention exhibiting excellent horizontal orientation gives a current density of twice or more the current density of an organic luminescent material exhibiting non-horizontal orientation.

Furthermore, the organic luminescent material of the present invention exhibiting excellent horizontal orientation allows, as shown in Fig. 2(a), an external quantum efficiency of 10% or higher to be stably obtained even in a low current density region of about $1\times10^{-3}$ [mA/cm$^2$].

On the contrary, it is understood that CBP exhibiting non-horizontal orientation is such that, as shown in Fig. 2(b), the external quantum efficiency (EQE, %) has high dependency on the current density.

That is, it is understood that the organic luminescent material of the present invention exhibiting excellent horizontal orientation has low dependency on the current density in a low current density region.

This phenomenon is explained in view of Figs. 3(a) to 3(f). Because since the organic luminescent material 10 of the present invention illustrated in Fig. 3(a) contains a predetermined donor-acceptor-type molecular structure 10' (10a, 10b, 10a) in its molecules, if the molecules are arranged in a layered form in the vertical direction, as shown in Fig. 3(d), a phenomenon of benzene-perfluorobenzene-type stacking occurs.

More specifically, when it is assumed that the molecules are disposed in the vertical direction, as shown on the left-hand side of the arrow in Fig. 3(c), the donor-like arylene groups (10a and 10a') of a lower molecule and the electron acceptor-like tetrafluoroarylene structure (10b) of an upper molecule come close to each other, and as shown in Fig. 3(d), the molecules are relatively shifted from each other in the horizontal direction to the extent of one arylene structure, while electrically pulling against each other in the vertical direction. Therefore, as shown on the right-hand side of the arrow in Fig. 3(c), the upper molecules are affected by the lower molecules that have already been aligned in the horizontal direction and are likely to be disposed in the horizontal direction.

Furthermore, as shown on the left-hand side of the arrow in Fig. 3(e), when it is assumed that the molecules are disposed in the vertical direction and there is a large number of upper molecules, it is speculated that even among the upper molecules, the arylene groups having donor-like properties and the electron acceptor-like tetrafluoroarylene structures of the upper molecules come close to each other and constitute a layered form.

Here, the stacking phenomenon of DPAPFP, which is a donor-acceptor-type molecule represented by the following formula (3), will be explained using a 3D molecular model obtained by X-ray crystal structure analysis, as illustrated in Fig. 13.

From Fig. 13, it is understood that when there are many DPAPFP molecules, in the diagram, the molecules are horizontally oriented in the transverse direction and are disposed in a layered form in the vertical direction, and particularly, tetrafluoroarylene groups and tetrahydroarylene groups approach close to each other in the vertical direction and are disposed in a layered form.

Then, as shown on the right-hand side of the arrow in Fig. 3(e), the molecules of the layered form resulting from these molecules may be more easily aligned in the horizontal direction. Moreover, once the molecules are aligned in the horizontal direction, it is difficult for them to be reoriented in the vertical direction.

That is, a molecule 10 having a predetermined donor-acceptor-type molecular structure 10' may be easily fixed in the horizontal direction, and as shown in Fig. 3(f), even when a dopant material or the like is present, the dopant material or the like may also be easily disposed in the horizontal direction. Therefore, it is concluded that charge transfer between the molecules is achieved smoothly, and satisfactory J-V characteristics are obtained.

On the contrary, the organic luminescent material exhibiting non-horizontal orientation (CBP) represented by the structural formula shown in Fig. 4(a) has a biphenylene structure 30' in the molecule, but this is not a predetermined donor-acceptor-type molecular structure. Therefore, the benzene-perfluorobenzene-type interaction described above does not occur, and as shown in Fig. 4(b), the group of molecules 30 is randomly oriented.

That is, even if the group of molecules is tentatively aligned in the horizontal direction with a dopant material or the like, since the fixing property in the vertical direction or in the horizontal direction is insufficient, it is reasonable to assume that movements in the vertical direction may occur.

In any case, if the organic luminescent material exhibits non-horizontal orientation, charge transfer between

the molecules is hindered, and it is speculated that the J-V characteristics are relatively inferior compared to the case of an organic luminescent material exhibiting horizontal orientation.

Furthermore, although not shown in the diagram, it is speculated that the organic luminescent material as a host material regulates to some extent the molecular arrangement of the material itself as well as the molecular arrangement of the dopant material contained in the light emitting layer, and jointly enhances the horizontal orientation of those molecules.

Therefore, when the organic luminescent material of the present invention as a host material is excited to emit light, the travelling direction of the light emission thus obtained is aligned with a predetermined direction (the normal direction of the thin film). That is, since the transition moments are aligned, higher light emission luminance may be obtained in a stable way even at a low current value.

2. Types of compounds

Regarding the types of compounds represented by the general formula (1), compounds represented by the following formulas (3) to (9) are mentioned as specific examples.

That is, it is preferable that the substituents $R^1$ to $R^4$ and a to h each independently represent a hydrogen atom or an alkyl group having 1 to 4 carbon atoms.

The reason is that, by using the compounds represented by the following formulas, organic luminescent materials which are relatively easily produced, are inexpensive, and have stable properties, may be obtained.

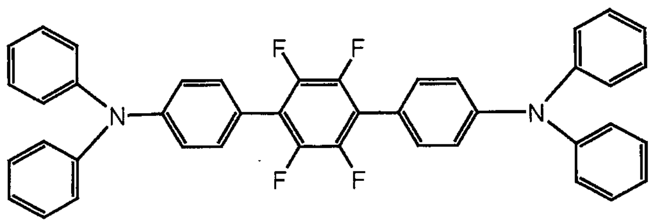

(3)

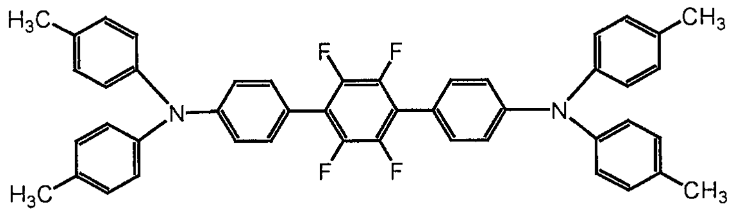

(4)

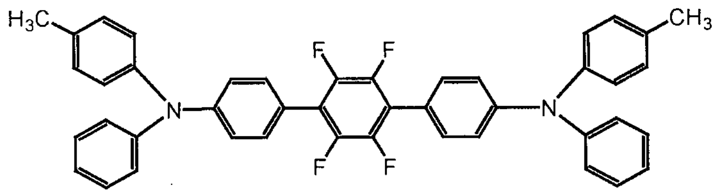

(5)

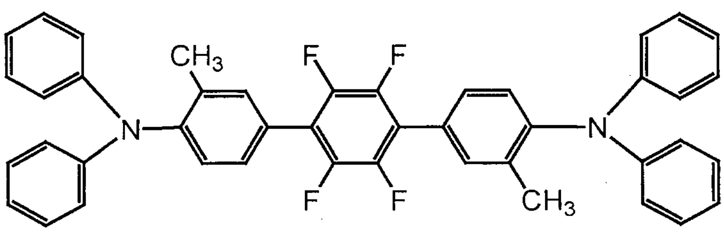

(6)

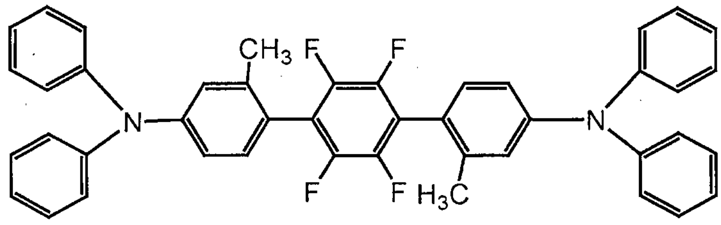

(7)

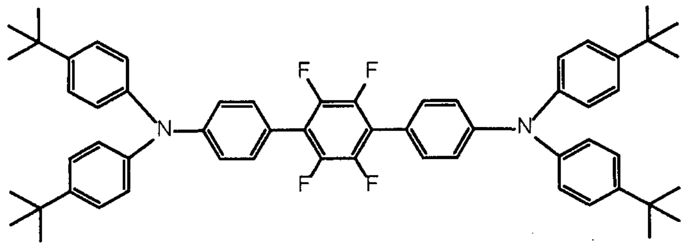

(8)

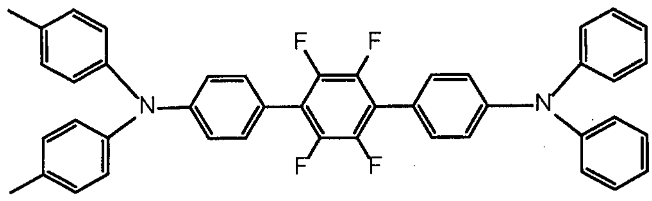

(9)

3. Horizontal orientation 1 (order parameter (S))

Furthermore, it may be said that the molecules of the organic luminescent material represented by the general formula (1) are arranged in the horizontal direction with respect to the base material surface when the material is produced into a film on the base material; that is, the organic luminescent material exhibits satisfactory horizontal orientation.

Here, whether the molecules of the organic luminescent material represented by the general formula (1) are arranged in the horizontal direction with respect to the base material surface may be determined based on the order parameter (S).

That is, the extinction coefficients (ko, ke) are actually measured using an ellipsometer, and then the order parameter may be calculated according to formula (1) that is disclosed in Example 1, described below.

Then, when such an order parameter has a value within the range of -0.5 to -0.1, the molecules of the organic luminescent material may be arranged substantially in the horizontal direction.

More specifically, this means that when the order parameter has a value of above -0.1, the horizontal orientation is insufficient and the J-V characteristics are not really satisfactory; on the other hand, when the order parameter is -0.5, the molecules are completely horizontally oriented.

Therefore, it is more preferable that such an order parameter has a value within the range of -0.5 to -0.2, and, even more preferably, a value within the range of -0.5 to - 0.22.

4. Horizontal orientation 2 (horizontal angle ($\theta 2$))

In addition, the horizontal orientation of an organic luminescent material used as a host material may be determined based on the horizontal angle ($\theta 2$) calculated from the order parameter (S).

That is, according to formula (1), that is disclosed in Example 1 described below, in a three-dimensional space formed by the XYZ-axes as shown in Fig. 3(b), the angle ($\theta$) formed by the Z-axis, which is a perpendicular axis, and the virtual axis line direction of the molecules of the organic luminescent material can be calculated.

Then, when an organic thin film made from an organic luminescent material is formed on a substrate, as illustrated in Fig. 3(b), the horizontal angle ($\theta 2$), which is the angle formed by the virtual axis line direction of the molecules of the organic luminescent material, is expressed as ($90° - \theta$). Therefore, the horizontal orientation of the organic luminescent material may be determined based on this horizontal angle.

More specifically, when such a horizontal angle is 31° or less, the molecules of the organic luminescent material used as a host material are arranged in a substantially horizontal direction, so that charge transfer is achieved smoothly, and the J-V characteristics become satisfactory.

However, if such a horizontal angle is excessively small, there may be excessive limitations on the type of organic luminescent material and the like that can be used.

Therefore, it is preferable to adjust such a horizontal angle to a value within the range of 1° to 27°, and it is more preferable to adjust it to a value within the range of 1° to 26°.

5. Luminescence quantum yield (Φ)

Furthermore, it is preferable that the luminescence quantum yield (Φ) measured for an organic luminescent material used as a host material has a value within the range of 30% to 80%.

The reason is that if such luminescence quantum yield has a value of below 30%, the emission luminance of the phosphorescent light thus obtained may be decreased, or it may be difficult to extract polarized components.

On the other hand, if such luminescence quantum yield has a value of above 80%, there may be excessive limitations on the type of organic luminescent material that can be used, or the like.

Therefore, it is more preferable to adjust the luminescence quantum yield that is measured using an organic luminescent material as a host material to a value within the range of 40% to 75%, and even more preferable to adjust it to a value within the range of 50% to 70%.

In addition, the luminescence quantum yield measured using an organic luminescent material as a host material may be measured according to the method that is described in Example 1 given below.

6. External quantum efficiency (EQE)

Furthermore, it is preferable that the external quantum efficiency (EQE) that is measured when a predetermined organic EL element is configured using an organic luminescent material as a host material has a value of 10% or higher at a current density within the range of 0.0005 to 10 $mA/cm^2$.

The reason is that, if such external quantum efficiency has a value of below 10%, the emission luminance thus obtainable may be excessively decreased.

On the other hand, if such external quantum efficiency has an excessively high value, for example, a value of above 20%, there may be excessive limitations on the type of dopant material that can be used, or the like.

Therefore, it is more preferable to adjust the external quantum efficiency that is measured using an organic luminescent material as a host material to a value within the range of 10.5% to 18%, and even more preferable to adjust it to a value within the range of 11% to 15%, in a predetermined range of the current density.

Meanwhile, the external quantum efficiency measured using an organic luminescent material as a host material, may be measured according to the method described in the Examples given below.

7. Weight average molecular weight

Furthermore, it is preferable that the weight average molecular weight of the organic luminescent material used as a host material has a value within the range of 400 to 1000.

The reason is that if such weight average molecular weight has a value of below 400, heat resistance or durability may be significantly decreased. On the other hand, if such weight average molecular weight has a value of above 1000, it may be difficult to uniformly disperse a predetermined guest material therein.

Therefore, it is more preferable to adjust the weight average molecular weight of the organic luminescent material used as a host material to a value within the range of 410 to 800, and even more preferable to adjust it to a value within the range of 420 to 600.

Meanwhile, such weight average molecular weight can be measured by, for example, the gel permeation chromatography (GPC) method based on calculations relative to polystyrene particle standards.

[Second embodiment]

A second embodiment of the present invention relates to a method for producing an organic luminescent material represented by the general formula (1), which has a donor-acceptor-type molecular structure containing an electron acceptor-like tetrafluoroarylene structure in its central part and a diphenylamine structure linked to each of the two ends of the tetrafluoroarylene structure through an electron donor-like arylene group, and which is used as a host material.

Also, the method is a method for producing an organic luminescent material that includes a first step of preparing a halogenated aryl formed from 1,4-dihalogenated tetrafluoroarylene; a second step of respectively preparing a first boronic acid ester formed from a para-aminoarylboronic acid ester having the substituents $R^1$ and $R^2$, and a second boronic acid ester formed from a para-aminoarylboronic acid ester having the substituents $R^3$ and $R^4$; and a third step

of cross-coupling the halogen atom at one end of the halogenated aryl and the first boronic acid ester under the action of a palladium catalyst and a basic nucleophile, and then cross-coupling the halogen atom at the other end of the halogenated aryl and the second boronic acid ester under the action of a palladium catalyst and a basic nucleophile.

Hereinafter, the method for producing a predetermined organic luminescent material, which is the second exemplary embodiment of the present invention, will be described specifically with appropriate reference to the drawings.

1. Scheme

The scheme is a method for producing an organic luminescent material represented by the general formula (1) described above, by cross-coupling a halogenated aryl and boronic acid esters, previously prepared in a first step and a second step, respectively, under the action of a palladium catalyst and a basic nucleophile.

That is, one of the advantages of this method is that an organic luminescent material represented by the general formula (1) described above may be produced in a short time and efficiently by using the Suzuki Miyaura Coupling method (SMC method), which aims at obtaining an asymmetric biaryl by cross-coupling an organoboron compound and a halogenated aryl under the action of a palladium catalyst and a basic nucleophile.

2. First step

The first step is a process of preparing a halogenated tetrafluoroaryl formed from 1,4-dihalogenated 2,3,5,6-tetrafluoroarylene prior to cross-coupling.

That is, the first step is a process of preparing at least one of 1,4-dibromo-2,3,5,6-tetrafluorobenzene, 1,4-dichloro-2,3,5,6-tetrafluorobenzene, 1,4-diiodo-2,3,5,6-tetrafluorobenzene and the like, as the predetermined halogenated tetrafluoroaryl.

Such a halogenated aryl may be produced according to a known synthesis method, or a commercially available product may be directly used.

3. Second step

The second step is a process of preparing predetermined boronic acid esters prior to cross-coupling.

That is, the second step is a process of preparing, respectively, a first boronic acid ester formed from a para-aminoarylboronic acid ester having the substituents $R^1$ and $R^2$ (the substituents $R^1$ and $R^2$ have the same definition as in the general formula (1)), and a para-aminoarylboronic acid ester having the substituents $R^3$ and $R^4$ (the substituents $R^3$ and $R^4$ have the same definition as in the general formula (1)).

Therefore, in order to obtain an organic luminescent material having an asymmetric structure, the substituents $R^3$ and $R^4$ must be different from the substituents $R^1$ and $R^2$; on the contrary, in order to obtain an organic luminescent material having a symmetric structure, the substituents $R^3$ and $R^4$ and the substituents $R^1$ and $R^2$ in the predetermined boronic acid esters must be identical (the same also applies to the substituents a to d and the substituents e to f). That is, in order to obtain an organic luminescent material having a symmetric structure, it is desirable to prepare any one of the first boronic acid ester or the second boronic acid ester and subject it to cross-coupling with a halogenated aryl as will be described below.

More specifically, as for the predetermined boronic acid ester, it is preferable to prepare, at least one of the following compounds: 1,4-{4-(diphenylamino)phenyl}-4,4,5,5-tetramethyl-1,3,2-dioxaborolane, 1,4-{4-(bis(4-methylphenyl)amino)phenyl}-4,4,5,5-tetramethyl-1,3,2-dioxaborolane, 1,4-{4-(4-methyl)(phenyl)amino)phenyl}-4,4,5,5-tetramethyl-1,3,2-dioxaborolane, 1,4-{4-(diphenylamino)-2-methylphenyl}-4,4,5,5-tetramethyl-1,3,2-dioxaborolane, 1,4-{4-(diphenylamino)-3-methylphenyl}-4,4,5,5-tetramethyl-1,3,2-dioxaborolane, 2-{4-(di-tert-butylphenylamino)phenyl-1-yl}-4,4,5,5-tetramethyl-1,3,2-dioxaborolane, and the like.

Meanwhile, such a boronic acid ester may be produced according to a known synthesis method, or a commercially available product may be directly used.

4. Third step

The third step is a process of obtaining an organic luminescent material represented by the general formula (1) described above, by sequentially cross-coupling a halogenated aryl represented by formula (8) (symbol X represents Br, Cl or I) and predetermined boronic acid esters represented by formula (9) and formula (10), which have been respectively prepared in the first step and the second step, as illustrated in the reaction formula (1) and reaction formula (2), respectively under the action of a palladium catalyst and a basic nucleophile.

Reaction Formula (1)

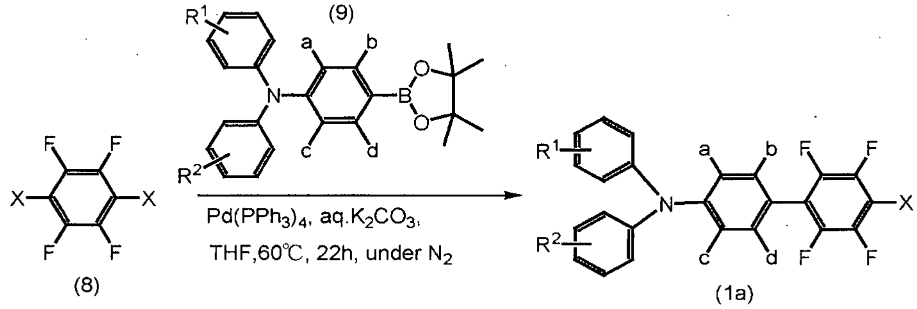


Reaction Formula (2)

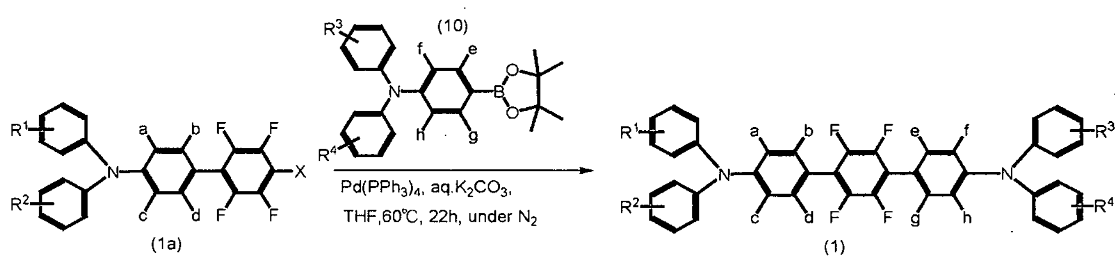


Here, in the third step, it is preferable to perform cross-coupling sequentially by adding dropwise the predetermined boronic acid esters represented by formula (9) and formula (10) respectively to the halogenated aryl represented by formula (8).

The reason is that, since the predetermined boronic acid esters may easily have their activity decreased in the reaction vessel, when the reaction is carried out by adding the esters dropwise, it is easier to maintain the activity of the predetermined boronic acid esters, and the yield of the reaction product may be dramatically increased.

More specifically, even a halogenated aryl that has strong electron acceptor-like properties and is generally considered to have very low reactivity may be allowed to effectively react with the predetermined boronic acid esters, and thus, the yield of the predetermined organic luminescent material may be dramatically increased. For example, it has been confirmed that the yield obtainable when the reactants are treated in batch without dropwise addition is about 1%; however, when the reaction is carried out by adding dropwise the boronic acid esters, the yield may be increased to a yield of 50% or higher.

Meanwhile, an example of adequate conditions, in the case of adding dropwise predetermined boronic acid esters and causing the esters to react with a predetermined halogenated aryl, is shown below.

Reaction temperature: 20°C to 80°C
Dropping time: 5 to 36 hours
Dropping rate: 0.05 to 0.4 molar equivalents/hour

In addition to that, when the organic luminescent material represented by the general formula (1) has an asymmetric structure in relation to the diarylamine containing the substituents $R^1$ and $R^2$ and a diarylamine containing the substituents $R^3$ and $R^4$, as illustrated in the reaction formula (1) and the reaction formula (2), first, any one of the boronic acid esters represented by formula (9) or formula (10) is subjected to a cross-coupling reaction, and subsequently, the other boronic acid ester is subjected to a cross-coupling reaction. Thus, the cross-coupling reaction is basically carried out in two stages.

On the other hand, when the organic luminescent material represented by the general formula (1) has a symmetric structure in relation to the diarylamine containing the substituents $R^1$ and $R^2$ and the diarylamine containing the substituents $R^3$ and $R^4$, similarly to the modification example described below, it is desirable to induce a cross-coupling reaction basically in one stage using any one of the predetermined boronic acid esters represented by formula (9) and formula (10).

5. Modified example

Furthermore, a modified example of production method is a method for producing an organic luminescent material having a symmetric structure represented by the general formula (1'), which has a donor-acceptor-type molecular

structure containing an electron acceptor-like tetrafluoroarylene structure in its central part and a diphenylamine structure linked to each of the two ends of the tetrafluoroarylene structure through an electron donor-like arylene group, and which is used as a host material.

Also, the method is a method for producing an organic luminescent material, the method including a first step of preparing a halogenated aryl formed from 1,4-dihalogenated tetrafluoroarylene; a second step of preparing a para-aminoarylboronic acid ester having the substituents $R^1$ and $R^2$ or the substituents $R^3$ and $R^4$; and a third step of cross-coupling the halogenated aryl and the boronic acid ester under the action of a palladium catalyst and a basic nucleophile.

That is, by carrying out the production method as such, a symmetric type organic luminescent material may be efficiently produced.

Furthermore, when the predetermined boronic acid ester is added dropwise to the halogenated aryl, even if the activation time of the boronic acid ester is short, a fresh boronic acid ester may be supplied all the time, and the reactivity to the halogenated aryl may be increased.

Therefore, with the dropping method, cross-coupling may be induced efficiently by using a highly active boronic acid ester, and thus a symmetric type organic luminescent material may be produced in a very short time.

[Third embodiment]

A third embodiment of the present invention relates to an organic light emitting element containing a light emitting layer which uses an organic luminescent material represented by the general formula (1), having a donor-acceptor-type molecular structure containing an electron acceptor-like tetrafluoroarylene structure in its central part and a diphenylamine structure linked to each of the two ends of the tetrafluoroarylene structure through an electron donor-like arylene group, and which is formed by incorporating a dopant material thereto.

The organic light emitting element is an organic light emitting element which includes, between a pair of electrodes composed of a positive electrode and a negative electrode, a light emitting layer or multiple organic thin film layers including a light emitting layer. The light emitting element is characterized in that its light emitting layer contains a dopant material and, as a host material that is the main component, the organic luminescent material of the first embodiment.

Hereinafter, the organic light emitting element (organic EL element), which is the third embodiment of the present invention, will be explained specifically with appropriate reference to the drawings.

1. Basic configuration

The organic light emitting element of the present invention, for example, a representative organic EL element 110 has, as illustrated in Fig. 5, a positive electrode 102 formed from a transparent conductive material, a hole transport layer 103 formed from a predetermined organic compound, a light emitting layer 104 formed from a predetermined organic compound, a hole blocking layer 105 formed from a predetermined organic compound, an electron transport layer 106 formed from a predetermined organic compound, and a negative electrode 107 formed from a metallic material, all laminated on a transparent substrate 101, which is a glass plate or the like.

That is, the organic EL element 110 is configured using such a multilayer structure as a basic configuration, and high luminance phosphorescence may be emitted through recombination of electrons and holes respectively injected from the electrodes.

Also, as illustrated in Fig. 6, in another structure of the organic EL element 111, an electron injection layer 107a, laminated as a thin film between the electron transport layer 106 and the negative electrode 107 is also included.

2. Light emitting layer

Furthermore, the light emitting layer contains the organic luminescent material of the first embodiment (host material), as well as a dopant material for the relevant host material.

Here, the kind of the dopant material is not particularly limited; however, since high luminance phosphorescence may be obtained in a more stable way over a long time by applying a relatively small electric current, it is preferable to use an iridium complex compound and a platinum complex compound, or any one thereof.

Whether phosphorescence is emitted or not may be determined on the basis of, for example, the luminescence lifetime in a phosphorescent light emitting material using a small-sized fluorescence lifetime analyzer, QUANTAURUS-TAU (manufactured by Hamamatsu Photonics K.K.).

That is, as illustrated in Fig. 7, the emission spectrum of phosphorescence is measured, and when a predetermined luminescence intensity ($Log_{10}$ (number of photons)) is maintained over a time of the order of the microsecond ($\mu$sec) or more, it may be determined that the luminescence thus obtainable is phosphorescence.

Here, specific examples of dopant materials such as iridium complex compounds are listed based on their emitted light color.

That is, examples of iridium complex compounds for extracting blue phosphorescence include bis[2-(4',6'-difluorophenyl)pyridinato-N,$C^{2'}$]iridium(III) tetrakis(1-pyrazolyl)borate, bis[2-(4',6'-difluorophenyl)pyridinato-N,$C^{2}$]iridium(III) picolinate, bis{2-[3',5'-bis(trifluoromethyl)phenyl]pyridinato-N,$C^{2'}$}iridium(III) picolinate, and bis[2-(4',6'-difluorophenyl)pyridinato-N,$C^{2'}$]iridium(III) acetylacetonate.

Examples of iridium complex compounds for extracting green phosphorescence include tris(2-phenylpyridinato-N,$C^{2'}$)iridium(III), bis(2-phenylpyridinato-N,$C^{2'}$)iridium(III) acetylacetonate, bis(1,2-diphenyl-1H-benzimidazolato)iridium(III) acetylacetonate, bis(benzo[h]quinolinato)iridium(III) acetylacetonate, and tris(benzo[h]quinolinato)iridium(III).

Examples of iridium complex compounds for extracting yellow phosphorescence include bis(2-phenylpyridinato-N,$C^{2'}$)(2-(3-(2-oxo-2H-chromenyl))pyridinato-N,$C^{4'}$)iridium(III), bis(2,4-diphenyl-1,3-oxazolato-N,$C^{2'}$)iridium(III) acetylacetonate, bis[2-(4'-perfluorophenylphenyl)pyridinato]iridium(III) acetylacetonate, bis(2-phenylbenzothiazolato-N,$C^{2'}$)iridium(III) acetylacetonate, (a-cetylacetonato)bis[2,3-bis(4-fluorophenyl)-5-methylpyrazinato]iridium(III), and (acetylacetonato)bis{2-(4-methoxyphenyl)-3,5-dimethylpyrazinato}iridium(III).

Examples of iridium complex compounds for extracting orange-colored phosphorescence include tris(2-phenylquinolinato-N,$C^{2'}$)iridium(III), bis(2-phenylquinolinato-N,$C^{2'}$)iridium(III) acetylacetonate, (acetylacetonato)bis(3,5-dimethyl-2-phenylpyrazinato)iridium(III), and (acetylacetonato)bis(5-isopropyl-3-methyl-2-phenylpyrazinato)iridium (III).

Furthermore, examples of iridium complex compounds for extracting red phosphorescence include bis[2-(2'-benzo[4,5-α]thienyl)pyridinato-N,$C^{3'}$]iridium(III) acetylacetonate, bis(1-phehylisoquinolinato-N,$C^{2'}$)iridium(III) acetylacetonate, (acetylacetonato)bis[2,3-bis(4-fluorophenyl)quinoxalinato]iridium(III), (acetylacetonato)is(2,3,5-triphenylpyridinato)iridium(III), and (dipivaloylmethanato)bis(2,3,5-triphenylpyrazinato)iridium(III).

Regarding platinum complex compounds, bis(2-phenylpyridinato-N,$C^{2'}$)platinum(II), (2-phenylpyridinato-N,$C^{2'}$)platinum(II) acetylacetonate, (N, N' - bis(salicylidene)ethylenediaminato-N,N',O,O')platinum(II), (tetraphenylporphynato-N,N',N",N"')platinum(II), and the like are preferably used singly or in combination of two or more kinds.

Furthermore, in regard to the type of dopant material, it is preferable to use a horizontally orientational material represented by the general formula (2). However, it has to be noted that, although iridium or platinum is also used in the horizontally orientational material represented by the general formula (2), it is assumed that the material is a complex compound other than the iridium complex compound or platinum complex compound described above.

Also, in particular, from the point of view of obtaining high quantum efficiency and high luminance phosphorescence in a stable way, phosphorescent light emitting materials represented by the following formulas (11) to (19) are more preferred.

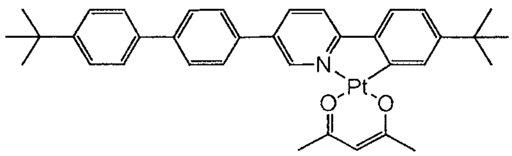
(11)

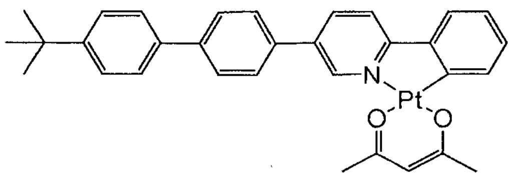
(12)

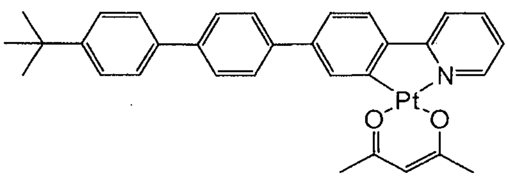
(13)

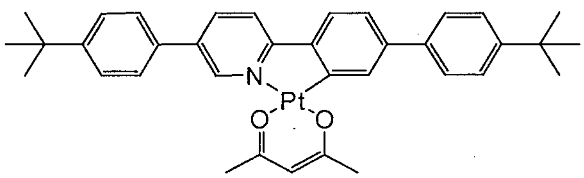
(14)

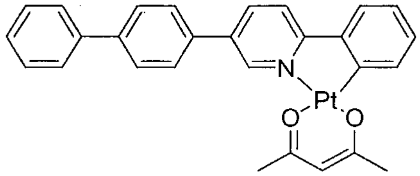

(15)

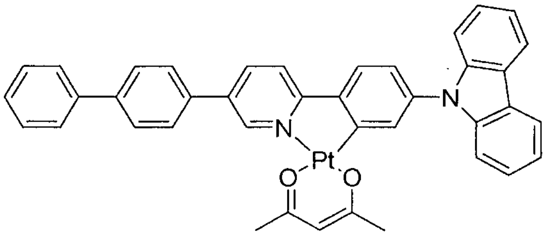

(16)

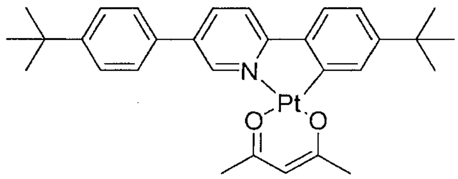

(17)

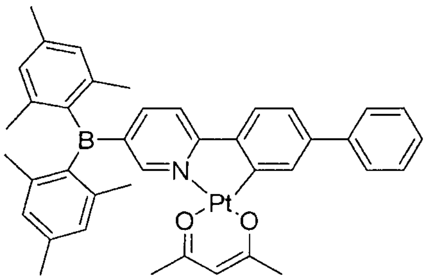

(18)

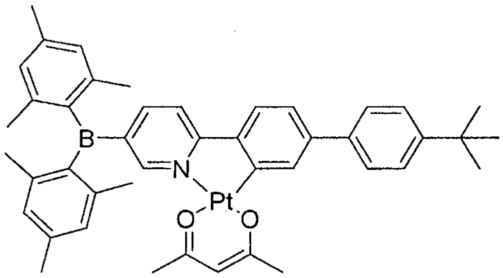

(19)

Furthermore, it is preferable that the incorporated amount of dopant material has a value within the range of 0.1% to 20% by weight, relative to the total amount (100% by weight) of the luminescent material, composed of the dopant material and a host material.

The reason is that, if the incorporated amount of such a dopant material has a value of below 0.1% by weight, the luminance of the emitted light may be significantly decreased.

On the other hand, if the amount of incorporation of such a dopant material has a value of above 20% by weight, it may be difficult for the dopant material to be uniformly dispersed in the predetermined host material, or crystallization may easily occur.

Therefore, it is more preferable to adjust the incorporated amount of dopant material to a value within the range of 1% to 10% by weight, and even more preferable to adjust it to a value within the range of 2% to 8% by weight, relative to the total amount of the luminescent material.

Furthermore, it is preferable that the weight average molecular weight of the dopant material has a value within the range of 400 to 1000.

The reason is that if such weight average molecular weight has a value of below 400, heat resistance or durability may be significantly decreased. On the other hand, if such weight average molecular weight has a value of above 1000,

it may be difficult to uniformly disperse the dopant material in the predetermined host material.

Therefore, it is more preferable to adjust the weight average molecular weight of the dopant material to a value within the range of 410 to 800, and even more preferably to a value within the range of 420 to 600.

Meanwhile, such a weight average molecular weight may be measured by, for example, the gel permeation chromatography (GPC) method based on calculations relative to polystyrene particle standards.

In addition to that, it is also preferable to add to the light emitting layer a material that helps electron transport. Examples of such an auxiliary electron transporting material include metal complexes of triazole derivatives, oxazole derivatives, polycyclic compounds, heteropolycyclic compounds such as bathocuproin, oxadiazole derivatives, fluorenone derivatives, diphenylquinone derivatives, thiopyran dioxide derivatives, anthraquinonedimethane derivatives, anthrone derivatives, carbodiimide derivatives, fluorenylidenemethane derivatives, distyrylpyrazine derivatives, acid anhydrides of aromatic cyclic tetracarboxylic acids such as naphthalene tetracarboxylic acid or perylene tetracarboxylic acid, phthalocyanine derivatives, and 8-quinolinol derivatives; metal phthalocyanines, various metal complexes represented by metal complexes having benzoxazole or benzothiazole as ligands; organic silane derivatives; and iridium complexes, which are used singly or in combination of two or more kinds.

3. Positive electrode

Furthermore, as the positive electrode, a metallic material or a metal oxide material having a relatively large work function, more specifically, a work function of 4 eV or more, is used.

Regarding such a metallic material or the like, for example, at least one material among indium tin oxide (ITO), indium zinc oxide (IZO), tin oxide ($SnO_2$), and zinc oxide (ZnO) is preferred.

Usually, it is preferable to adjust the thickness of the positive electrode to a value within the range of 300 to 3000 angstroms.

4. Negative electrode

Furthermore, for the negative electrode, a metallic material or a metal oxide material having a relatively small work function, more specifically, a work function of below 4 eV, is used.

Preferred examples of such a metallic material or the like include lithium, barium, aluminum, magnesium, indium, silver, and alloys of the respective metals.

Usually, it is preferable to adjust the thickness of the negative electrode to a value within the range of 100 to 5000 angstroms.

Meanwhile, if any one of the positive electrode or the negative electrode described above is transparent or semitransparent, such as in the case of indium tin oxide (ITO), the predetermined phosphorescence may be extracted to the outside.

5. Electron transport layer

As illustrated in Fig. 5 and Fig. 6, it is preferable for the organic luminescent element to include at least the light emitting layer 104, the positive electrode 102 and the negative electrode 107 described above, and a hole blocking layer 105 that will be described below, and to provide an electron transport layer 106 at a predetermined position.

Here, examples of the electron transporting material that is incorporated into the electron transport layer include metal complexes of triazole derivatives, oxazole derivatives, polycyclic compounds, heteropolycyclic compounds such as bathocuproin, oxadiazole derivatives, fluorenone derivatives, diphenylquinone derivatives, thiopyran dioxide derivatives, anthraquinonedimethane derivatives, anthrone derivatives, carbodiimide derivatives, fluorenylidenemethane derivatives, distyrylpyrazine derivatives, acid anhydrides of aromatic cyclic tetracarboxylic acids such as naphthalene tetracarboxylic acid or perylene tetracarboxylic acid, phthalocyanine derivatives, and 8-quinolinol derivatives; metal phthalocyanines; various metal complexes represented by metal complexes having benzoxazole or benzothiazole as ligands; organic silane derivatives; and iridium complexes, which are used singly or in combination of two or more kinds.

6. Hole blocking layer

Furthermore, as illustrated in Fig. 5 and Fig. 6, it is preferable to provide a hole blocking layer 105.

It is because when the hole blocking layer 105 is provided as such, the luminescence efficiency may be increased, and also, the lifetime of the organic EL element may also be lengthened.

Here, the hole blocking layer 105 may be provided using the electron transporting materials described above, and it is preferable to prepare a mixed layer in which two or more kinds of electron transporting materials are mixed and laminated by co-vapor deposition or the like.

It is also preferable for the electron transporting materials contained in the hole blocking layer to have an ionization potential higher than that of the ionization potential of the light emitting layer.

7. Others

Although not shown in the diagram, it is preferable to seal the periphery of the display region of the organic EL element using an epoxy resin, an acrylic resin or the like and also using a predetermined member, in order to eliminate the influence of moisture and to increase durability.
It is also preferable to inject an inert gas such as nitrogen or argon, or an inert liquid such as a fluorinated hydrocarbon or a silicone oil into the gap between the display region of the organic EL element and the predetermined member.
On the other hand, it is also preferable to draw a vacuum in the gap or to encapsulate a hygroscopic compound in such a gap, so that the influence of moisture may be eliminated.

EXAMPLES

Hereinafter, the present invention is explained in more detail with reference to the Examples.

[Example 1]

1. Production of an organic luminescent material

First, 1,4-bis{4-(diphenylamino)phenyl}-2,3,5,6-tetrafluorobenzene (hereinafter, may be referred to as DPAPFP) represented by formula (3) was synthesized from 1,4-dibromo-2,3,5,6-tetrafluorobenzene.
That is, 1,4-dibromo-2,3,5,6-tetrafluorobenzene (a commercially available product, 608 mg, 2 mmol) as a raw material compound was placed in a container attached to a dropping apparatus and a stirring apparatus, and then tetrakis(triphenylphosphine)palladium(0) (200 mg, 0.17 mmol), potassium carbonate (2.54 mg, 18 mmol), 50 ml of tetrahydrofuran (THF), and 12 ml of water were added thereto in a nitrogen atmosphere. The mixture was degassed and then heated to 60°C.
Next, a liquid substance obtained by dissolving 1,4-{4-(diphenylamino)phenyl}-4,4,5,5-tetramethyl-1,3,2-dioxaborolane (commercially available product, 1485 mg, 4 mmol) in 10 ml of THF and degassing the solution, was added dropwise thereto over 12 hours. Furthermore, the mixture was heated and stirred under the conditions of 60°C and 22 hours, and thus a reaction solution was obtained.
Next, the solvent from the reaction solution thus obtained was evaporated to dryness (evaporated), and the resulting residual solid was collected by filtration. Furthermore, the residual solid was dissolved in dichloromethane, and the solution was washed with water and saturated brine and dried over magnesium sulfate. Subsequently, the solvent was evaporated to dryness (evaporated), and the resulting residual solid was collected by filtration.
Finally, the residual solid thus obtained was subjected to purification by silica gel column chromatography, and recrystallization using dichloromethane/hexane. Thus, 1,4-bis{4-(diphenylamino)phenyl}-2,3,5,6-tetrafluorobenzene represented by formula (3) (DPAPFP, 675 mg, 1.06 mmol, yield 53%) was obtained in a white powder form.

2. Formation of a thin film based on the organic luminescent material thus obtained

A thin film (thickness: 50 nm) made of the organic luminescent material (DPAPFP) thus obtained was formed on a silicon substrate (size: 10 mm × 10 mm × 0.7 mm) using a vacuum deposition method.
The vapor deposition conditions were as follows.
Film forming apparatus: Super precision alignment mechanism-based vapor deposition apparatus, E-180-S (manufactured by ALS Technology Co., Ltd.)
Film forming speed: 1.0 Å/sec
Film forming pressure: $2.0 \times 10^{-4}$ Pa
Film forming time: 9.3 minutes

3. Evaluation of the organic luminescent material

(1) Evaluation of the order parameter (S)

For the thin film made from the organic luminescent material (DPAPFP) obtained on the silicon substrate, by making use of an ellipsometer (M-2000 manufactured by J.A. Woollam Co.), the amplitude ratio and the phase difference

(Psi and Delta) of a polarized light that enters at various angles with respect to the substrate were measured by the change in state of the reflected polarized light. Based on these values, a presumable optical model was established, and also, the extinction coefficient and the like were calculated by performing a fit such that the mean square errors of the two values would be minimal. Thus, the order parameter (S) was determined according to the following mathematical formula (1). The results thus obtained are presented in Table 1.

In addition, the construction of an optical model, the fit of the optical model and the measured value for minimizing the mean square errors, and the like were carried out using a software program for ellipsometry data analysis, WASE32 (manufactured by J.A. Woollam Co.).

$$S = \frac{k_e - k_o}{k_e + 2k_o} = \frac{1}{2}\left\langle 3\cos^2\theta - 1 \right\rangle \quad (1)$$

Here, in mathematical formula (1), the symbol k represents the extinction coefficient; the subscripts o and e stand for the extinction coefficients in the xy-direction (planar direction) and the z-direction (vertical direction), respectively, with respect to the substrate. Then, making use of the ellipsometer mentioned above, the extinction coefficient (k) of the organic luminescent material (DPAPFP) thus obtained can be measured. In Fig. 8, the extinction coefficients are respectively shown by presenting the extinction coefficient chart thus obtained on the vertical axis and the wavelength on the horizontal axis.

In addition to that, in a three-dimensional space formed by the XYZ-axes, $\theta$ in formula (1) is defined as the angle formed by the Z-axis, which is the vertical axis, and the virtual axis line direction of the molecules of the organic luminescent material.

(2) Evaluation of the horizontal orientation

Furthermore, the angle ($\theta$) formed by the Z-axis, which is the vertical axis, and the virtual axis line direction of the molecules of the organic luminescent material was calculated from the value of the order parameter (S) mentioned above. Also, as an indicator of horizontal orientation, the horizontal angle ($\theta 2 = 90° - \theta$), that is, the angle formed between the substrate and the virtual axis line direction of the molecules of the organic luminescent materials (DPAPFP), was calculated. The results thus obtained are presented in Table 1.

(3) Luminescence quantum yield

For the thin film of the organic luminescent material (DPAPFP) thus obtained, the luminescence quantum yield (internal quantum efficiency) at a predetermined wavelength (337 nm) was measured using an absolute PL quantum yield measurement apparatus (QUANTAURUS-QY C11347-01, manufactured by Hamamatsu Photonics Co., Ltd.). The results thus obtained are presented in Table 1.

(4) NMR

NMR (nuclear magnetic resonance) was performed with a JNM-EPC400 apparatus (manufactured by JEOL, Ltd.) on the obtained organic luminescent material (DPAPFP), previously dissolved in deuterated chloroform solvent. The NMR chart thus obtained is presented in Fig. 9.

(5) FT-IR

The FT-IR chart of the obtained organic luminescent material (DPAPFP) was measured using the KBr tablet method using an FT/IR-6100 (manufactured by JASCO Corp.). The FT-IR chart thus obtained is presented in Fig. 10.

(6) Light absorption wavelength spectrum and light emission peaks

The light absorption wavelength spectrum of the obtained organic luminescent material (DPAPFP) was measured using an ultraviolet/visible spectrophotometer, UV-2550 (manufactured by Shimadzu Corp.).

The luminescence intensity (fluorescence emission spectrum) in the organic luminescent material (DPAPFP) thus obtained was measured using a fluorescence spectrophotometer, FP-6500 (manufactured by JASCO Corp.).

The light absorption wavelength spectrum thus obtained is presented in Fig. 11, and the fluorescence emission spectrum thus obtained is presented in Fig. 12.

(7) J-V characteristics

The following organic EL element was configured using the organic luminescent material (DPAPFP) thus obtained. Next, the J-V characteristics (current density vs. voltage) were measured. The J-V characteristics curve thus obtained is presented in Fig. 1 as line A. Furthermore, the current densities at representative voltages (4 V, 4.5 V, and 5 V) are presented in Table 1.

(Configuration of the organic EL element)

ITO (100 nm)/TPD (50 nm)/6 wt% $Ir(ppy)_2Pc$-DPAPFP (20 nm)/TPBi (50 nm)/LiF (0.5 nm)/Al (100 nm)
That is, a glass substrate (12 mm in length × 12 mm in width × 1 mm in thickness), on which an indium tin oxide film of 100 nm thickness with the function of a positive electrode had been deposited, was prepared.
On the indium tin oxide film, were respectively laminated: an N,N'-bis(3-methylphenyl)-N,N'-bis(phenyl)benzidine deposit layer (TPD, 50 nm) as a hole transport layer; a DPAPFP deposit layer (20 nm) containing (bis(2-phenylpyridinato-N,$C^{2'}$) (2- (3- (2-oxo-2H-chromenyl))pyridinato-N,$C^{4'}$)iridium(III) (hereinafter, may be referred to as $Ir(ppy)_2Pc$) at a concentration of 6% by weight as a light emitting layer; a 2,2',2''-(1,3,5-benzenetolyl)-tris(1-phenyl-1-H-benzimidazole) deposit layer (TPBi, 50 nm) as an electron transport layer; an LiF deposit layer (0.1 nm) as a negative electrode; and an Al deposit layer (100 nm). A power supply was connected thereto and an organic EL element was thus obtained.
The vapor deposition conditions were as follows.
Film forming apparatus: Super precision alignment mechanism-based vapor deposition apparatus, E-180-S (manufactured by ALS Technology Co., Ltd.)
Film forming speed: host 1.0 Å/sec, dopant 0.007 Å/sec
Film forming pressure: $2.0\times10^{-4}$ Pa
Film forming time: 3.7 minutes

(8) External quantum efficiency

An organic EL element similar to that used for measuring the J-V characteristics was configured, and its external quantum efficiency was measured. That is, the external quantum efficiency thus obtained was reported as a function of the current density. The characteristic curve thus obtained is presented in Fig. 2(a). Also, the external quantum efficiencies at representative current densities ($1\times10^{-3}$ mA/cm$^2$, $1\times10^{-1}$ mA/cm$^2$, and $1\times10^{1}$ mA/cm$^2$) are presented in Table 1.

[Example 2]

In Example 2, 1,4-bis{4-(dimethylphenylamino)phenyl-1-yl}-2,3,5,6-tetrafluorobenzene represented by formula (4) was synthesized as an organic luminescent material, and the material was evaluated in the same way as in Example 1.
Therefore, in regard to the organic luminescent material thus obtained, Fig. 14(a) presents the NMR chart; Fig. 14(b) presents the FT-IR chart; Fig 15(a) shows diagram representing the anisotropy of the extinction coefficient (k) as a function of the wavelength ($\lambda$); Fig. 15(b) presents the light absorption wavelength spectrum; and Fig. 15(c) presents the fluorescence emission spectrum.
First, 1,4-bis{4-(dimethylphenylamino)phenyl-1-yl}-2,3,5,6-tetrafluorobenzene represented by formula (4) was synthesized from 1,4-dibromo-2,3,5,6-tetrafluorobenzene.
That is, 1,4-dibromo-2,3,5,6-tetrafluorobenzene (a commercially available product, 616 mg, 2 mmol) as a raw material compound was placed in a container attached to a dropping apparatus and a stirring apparatus, and then tetrakis(triphenylphosphine)palladium(0) (200 mg, 0.17 mmol), potassium carbonate (2.54 mg, 18 mmol), 50 ml of tetrahydrofuran (THF), and 12 ml of water were added thereto in a nitrogen atmosphere. The mixture was degassed and then heated to 60°C.
Next, a liquid substance obtained by dissolving 2-{4-(dimethylphenylamino)phenyl-1-yl}-4,4,5,5-tetramethyl-1,3,2-dioxaborolane (1,597 mg, 4 mmol) in 10 ml of THF and degassing the solution, was added dropwise thereto over 12 hours. Furthermore, the mixture was heated and stirred under the conditions of 60°C and 72 hours, and thus a reaction solution was obtained.
Next, the solvent from the reaction solution thus obtained was evaporated to dryness (evaporated), and the resulting residual solid was collected by filtration. Furthermore, the residual solid was dissolved in dichloromethane, and the solution was washed with water and saturated brine and dried over magnesium sulfate. Subsequently, the solvent was evaporated to dryness (evaporated), and the resulting residual solid was collected by filtration.
Finally, the residual solid thus obtained was subjected to purification by silica gel column chromatography, and recrystallization using dichloromethane/hexane. Thus, 1,4-bis{4-(dimethylphenylamino)phenyl-1-yl}-2,3,5,6-tetrafluorobenzene represented by formula (4) (881 mg, 1.27 mmol, yield 63%) was obtained in a white powder form.

[Example 3]

In Example 3, 1,4-bis{4-(di-tert-butylphenylamino)phenyl-1-yl}-2,3,5,6-tetrafluorobenzene represented by formula (8) was synthesized as an organic luminescent material, and the material was evaluated in the same way as in Example 1.

Therefore, in regard to the organic luminescent material thus obtained, Fig. 16(a) presents the NMR chart; Fig. 16(b) presents the FT-IR chart; Fig 17(a) shows the diagram representing the anisotropy of the extinction coefficient (k) as a function of the wavelength ($\lambda$); Fig. 17(b) presents the light absorption wavelength spectrum; and Fig. 17(c) presents the fluorescence emission spectrum.

First, 1,4-bis{4-(di-tert-butylphenylamino)phenyl-1-yl}-2,3,5,6-tetrafluorobenzene represented by formula (8) was synthesized from 1,4-dibromo-2,3,5,6-tetrafluorobenzene.

That is, 1,4-dibromo-2,3,5,6-tetrafluorobenzene (commercially available product, 462 mg, 1.5 mmol) as a raw material compound was placed in a container attached to a dropping apparatus and a stirring apparatus, and then tetrakis(triphenylphosphine)palladium(0) (150 mg, 0.13 mmol), potassium carbonate (1.91 mg, 13.5 mmol), 30 ml of tetrahydrofuran (THF), and 9 ml of water were added thereto in a nitrogen atmosphere. The mixture was degassed and then heated to 60°C.

Next, a liquid substance obtained by dissolving 2-{4-(di-tert-butylphenylamino)phenyl-1-yl}-4,4,5,5-tetramethyl-1,3,2-dioxaborolane (1,450 mg, 3 mmol) in 15 ml of THF and degassing the solution, was added dropwise thereto over 12 hours. Furthermore, the mixture was heated and stirred under the conditions of 60°C and 48 hours, and thus a reaction solution was obtained.

Next, the solvent from the reaction solution thus obtained was evaporated to dryness (evaporated), and the resulting residual solid was collected by filtration. Furthermore, the residual solid was dissolved in dichloromethane, and the solution was washed with water and saturated brine and dried over magnesium sulfate. Subsequently, the solvent was evaporated to dryness (evaporated), and the resulting residual solid was collected by filtration.

Finally, the residual solid thus obtained was subjected to purification by silica gel column chromatography, and recrystallization using dichloromethane/hexane. Thus, 1,4-bis{4-(di-tert-butylphenylamino)phenyl-1-yl}-2,3,5,6-tetrafluorobenzene represented by formula (8) (1,013 mg, 1.18 mmol, yield 79%) was obtained in a white powder form.

[Example 4]

In Example 4, 1-{4-(diphenylamino)phenyl-1-yl}-4-{4-(dimethylpheylamino)phenyl-1-yl}-2,3,5,6-tetrafluorobenzene represented by formula (9) was synthesized as the organic luminescent material, and the material was evaluated in the same way as in Example 1.

Therefore, in regard to the organic luminescent material thus obtained, Fig. 18(a) presents the NMR chart; Fig. 18(b) presents the FT-IR chart; Fig 19(a) shows the diagram representing the anisotropy of the extinction coefficient (k) as a function of the wavelength ($\lambda$); Fig. 19(b) presents the light absorption wavelength spectrum; and Fig. 19(c) presents the fluorescence emission spectrum.

First, 1-{4-(diphenylamino)phenyl-1-yl}-4-{4-(dimethylphenylamino)phenyl-1-yl}-2,3,5,6-tetrafluorobenzene represented by formula (9) was synthesized from 1,4-dibromo-2,3,5,6-tetrafluorobenzene.

That is, 1-{4-(diphenylamino)phenyl-1-yl}-4-bromo-2,3,5,6-tetrafluorobenzene (473 mg, 1 mmol) as a raw material compound was placed in a container attached to a dropping apparatus and a stirring apparatus, and then tetrakis(triphenylphosphine)palladium(0) (100 mg, 0.09 mmol), potassium carbonate (1.27 mg, 9.0 mmol), 25 ml of tetrahydrofuran (THF), and 6 ml of water were added thereto in a nitrogen atmosphere. The mixture was degassed and then heated to 60°C.

Next, a liquid substance obtained by dissolving 2-{4-(dimethylphenylamino)phenyl-1-yl}-4,4,5,5-tetramethyl-1,3,2-dioxaborolane (423 mg, 1 mmol) in 15 ml of THF and degassing the solution, was added dropwise thereto over 12 hours. Furthermore, the mixture was heated and stirred under the conditions of 60°C and 15 hours, and thus a reaction solution was obtained.

Next, the solvent from the reaction solution thus obtained was evaporated to dryness (evaporated), and the resulting residual solid thus produced was collected by filtration. Furthermore, the residual solid was dissolved in dichloromethane, and the solution was washed with water and saturated brine and dried over magnesium sulfate. Subsequently, the solvent was evaporated to dryness (evaporated), and the resulting residual solid was collected by filtration.

Finally, the residual solid thus obtained was subjected to purification by silica gel column chromatography, and recrystallization using dichloromethane/hexane. Thus, 1-{4-(diphenylamino)phenyl-1-yl}-4-{4-(dimethylphenylamino)phenyl-1-yl}-2,3,5,6-tetrafluorobenzene represented by formula (9) (221 mg, 0.33 mmol, yield 33%) was obtained as a white powder.

[Comparative Example 1]

In Comparative Example 1, 4,4'-di(N-carbazolyl)biphenyl (may be referred to as CBP) represented by formula (20) was used as the host material, and the order parameter (S) and the like were evaluated, or after an organic EL element was configured, the J-V characteristics, the external quantum efficiency and the like were evaluated, in the same way as in Example 1.

The J-V characteristics curve thus obtained is presented in Fig. 1 as line B, and the external quantum efficiency thus obtained is presented in Fig. 2(b) as a function of the current density.

The current densities at representative voltages (4 V, 4.5 V, and 5 V), and the external quantum efficiencies at representative current densities ($1\times10^{-3}$ mA/cm$^2$, $1\times10^{-1}$ mA/cm$^2$, and $1\times10^{1}$ mA/cm$^2$) are respectively presented in Table 1.

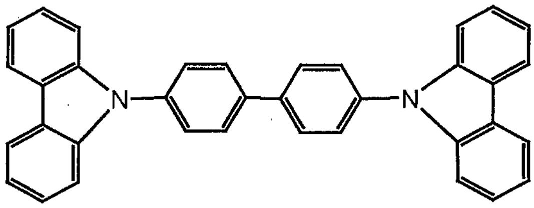

(20)

[Table 1]

| | | Example 1 | Example 2 | Example 3 | Example 4 | Comparative Example 1 |
|---|---|---|---|---|---|---|
| Order parameter (S) | | -0.34 | -0.46 | -0.43 | -0.39 | 0.1 |
| $\theta$ (°) | | 71.0 | 81.0 | 77.2 | 74.5 | Random |
| Horizontal angle ($\theta$2) (°) | | 19 | 9 | 12.8 | 15.5 | Random |
| Luminescence quantum yield (%) | | 78.1 | 75.3 | 67 | 84.9 | 66 |
| J-V characteristics (mA/cm$^2$ ) | 4.0 V | 3.0 | | | | 0.8 |
| | 4.5 V | 7.0 | | | | 3.0 |
| | 5.0 V | 15.0 | | | | 8.0 |
| EQE (%) | $1\times10^{-3}$ (mA/cm$^2$) | 10.0 | | | | 6.2 |
| | $1\times10^{-1}$ (mA/cm$^2$) | 11.8 | | | | 15.9 |
| | $1\times10^{-1}$ (mA/cm$^2$) | 11.0 | | | | 12.6 |

In Examples 1 to 4, all of the order parameters had values of -0.34 or less, and the horizontal angles had values of 19° or less. Thus, organic luminescent materials with excellent horizontal orientation were obtained.

Furthermore, in Example 4 in which an organic luminescent material presenting an asymmetric molecular structure was used, a high luminescence quantum efficiency of 80% or higher was obtained.

On the other hand, the compound of Comparative Example 1 had an order parameter of 0.1, and its molecules were randomly oriented. Thus, the compound exhibited non-horizontal orientation.

INDUSTRIAL APPLICABILITY

Thus, as described above, according to the present invention, the following may be obtained: an organic luminescent material used as a host material which exhibits excellent horizontal orientation or the like when produced into a film; an efficient method for producing such an organic luminescent material; and an organic EL element, (phosphorescent light emitting element) which gives a relatively high electric current value even if a low voltage is applied and exhibits high external quantum efficiency (EQE) by applying a small electric current.

Furthermore, according to the method for producing an organic luminescent material of the present invention, particularly, in the third step, while the boronic acid esters are added dropwise to the halogenated aryl, by being subjected to cross-coupling, they may be used in a fresh state. Thus, even for a halogenated aryl which is said to have low reactivity, the reaction yield may usually be dramatically increased.

EXPLANATIONS OF LETTERS OR NUMERALS

10: HOST MATERIAL (MOLECULES OF HOST MATERIAL)
10': DONOR-ACCEPTOR-TYPE MOLECULAR STRUCTURE
12: SUBSTRATE (GLASS SUBSTRATE)
20: DOPANT MATERIAL
30: NON-HORIZONTALLY ORIENTATIONAL HOST MATERIAL (MOLECULES OF HOST MATERIAL)
110, 111: ORGANIC EL ELEMENT
101: TRANSPARENT SUBSTRATE
102: POSITIVE ELECTRODE
103: HOLE TRANSPORT LAYER
104: LIGHT EMITTING LAYER
105: HOLE BLOCKING LAYER
106: ELECTRON TRANSPORT LAYER
107: NEGATIVE ELECTRODE
107a: ELECTRON INJECTION LAYER

## Claims

1. An organic luminescent material represented by the following general formula (1), having a donor-acceptor-type molecular structure containing an electron acceptor-like tetrafluoroarylene structure in its central part and a diphenylamine structure linked to each of the two ends of the tetrafluoroarylene structure through an electron donor-like arylene group, and being used as a host material;

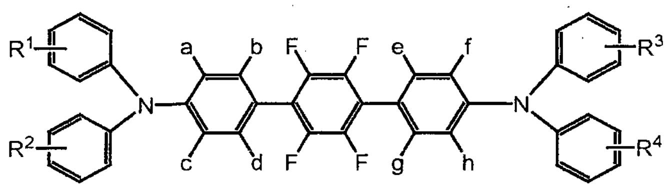

(1)

in the general formula (1), the substituents $R^1$ to $R^4$ and a to h each independently represent a hydrogen atom, an alkyl group having 1 to 20 carbon atoms, a substituted alkyl group having 1 to 20 carbon atoms, an aryl group having 6 to 20 carbon atoms, a substituted aryl group having 6 to 20 carbon atoms, or an amino group.

2. The organic luminescent material according to claim 1, wherein the substituents $R^1$ to $R^4$ and a to h each independently represent a hydrogen atom or an alkyl group having 1 to 4 carbon atoms.

3. The organic luminescent material according to claim 1 or 2, wherein the order parameter calculated from the anisotropy of the extinction coefficient has a value within the range of -0.5 to -0.1.

4. The organic luminescent material according to any one of claims 1 to 3, wherein in a three-dimensional space formed by the XYZ-axes, when the angle formed by the Z-axis, which is the vertical axis, and the virtual axis line direction of the molecules of the organic luminescent material is designated as $\theta$, the horizontal angle ($\theta 2$) represented by ($90° - \theta$) has a value of 31° or less.

5. A method for producing an organic luminescent material represented by the following general formula (1), which has a donor-acceptor-type molecular structure containing an electron acceptor-like tetrafluoroarylene structure in

its central part and a diphenylamine structure linked to each of the two ends of the tetrafluoroarylene structure through an electron donor-like arylene group, and which is used as a host material,
the method comprising:

a first step of preparing a halogenated aryl formed from 1,4-dihalogenated tetrafluoroarylene;
a second step of respectively preparing a first boronic acid ester formed from a para-aminoarylboronic acid ester having the substituents $R^1$ and $R^2$, and a second boronic acid ester formed from a para-aminoarylboronic acid ester having the substituents $R^3$ and $R^4$; and
a third step of cross-coupling the halogen atom at one end of the halogenated aryl and the first boronic acid ester under the action of a palladium catalyst and a basic nucleophile, and then cross-coupling the halogen atom at the other end of the halogenated aryl and the second boronic acid ester under the action of a palladium catalyst and a basic nucleophile;

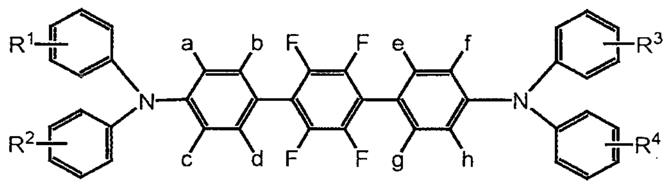
(1)

in the general formula (1), the substituents $R^1$ to $R^4$ and a to h each independently represent a hydrogen atom, an alkyl group having 1 to 20 carbon atoms, a substituted alkyl group having 1 to 20 carbon atoms, an aryl group having 6 to 20 carbon atoms, a substituted aryl group having 6 to 20 carbon atoms, or an amino group.

**6.** The method for producing an organic luminescent material according to claim 5, wherein, in the third step, the halogenated aryl is subjected to cross-coupling while the first boronic acid ester and the second boronic acid ester are respectively added dropwise thereto.

**7.** A method for producing an organic luminescent material represented by the following general formula (1'), which has a donor-acceptor-type molecular structure containing an electron acceptor-like tetrafluoroarylene structure in its central part and a diphenylamine structure linked to each of the two ends of the tetrafluoroarylene structure through an electron donor-like arylene group, and which is used as a host material,
the method comprising:

a first step of preparing a halogenated aryl formed from 1,4-dihalogenated tetrafluoroarylene;
a second step of preparing a boronic acid ester formed from a para-aminoarylboronic acid ester having the substituents $R^1$ and $R^2$ or the substituents $R^3$ and $R^4$; and
a third step of cross-coupling the halogenated aryl and the boronic acid ester under the action of a palladium catalyst and a basic nucleophile;

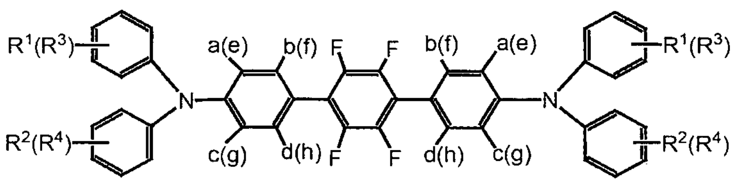
(1')

in the general formula (1'), the substituents $R^1$ and $R^2$ (or $R^3$ and $R^4$) and a to d (or e to h) each independently represent a hydrogen atom, an alkyl group having 1 to 20 carbon atoms, a substituted alkyl group having 1 to 20 carbon atoms, an aryl group having 6 to 20 carbon atoms, a substituted aryl group having 6 to 20 carbon atoms, or an amino group.

**8.** The method for producing an organic luminescent material according to claim 7, wherein, in the third step, the halogenated aryl is subjected to cross-coupling while the boronic acid ester is added dropwise thereto.

**9.** An organic light emitting element comprising a light emitting layer which uses, as a host material, an organic luminescent material represented by the following general formula (1), the organic luminescent material having a donor-acceptor-type molecular structure containing an electron acceptor-like tetrafluoroarylene structure in its central part and a diphenylamine structure linked to each of the two ends of the tetrafluoroarylene structure through an electron donor-like arylene group, and is formed by incorporating a dopant material thereto;

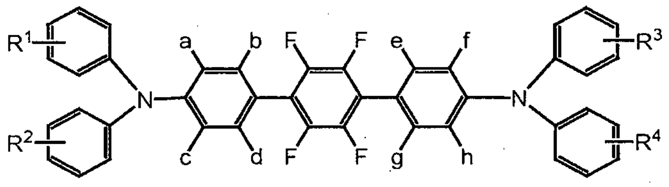
(1)

in the general formula (1), the substituents $R^1$ to $R^4$ and a to h each independently represent a hydrogen atom, an alkyl group having 1 to 20 carbon atoms, a substituted alkyl group having 1 to 20 carbon atoms, an aryl group having 6 to 20 carbon atoms, a substituted aryl group having 6 to 20 carbon atoms, or an amino group.

**10.** The organic light emitting element according to claim 9, wherein the dopant material is an iridium complex compound and a platinum complex compound, or any one of the complex compounds.

**11.** The organic light emitting element according to claim 9, wherein the dopant material is a horizontally orientational compound represented by the following general formula (2), having a straight-chained conjugated structure, a 2-phenylpyridine ligand, a coordinating metal, and an acetylacetonate ligand in the molecule;

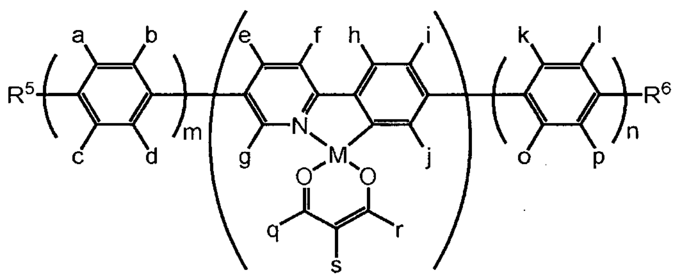
(2)

in the general formula (2), $R^5$ and $R^6$ each independently represent an alkyl group having 1 to 20 carbon atoms, a substituted alkyl group having 1 to 20 carbon atoms, an aryl group having 6 to 20 carbon atoms, a substituted aryl group having 6 to 20 carbon atoms, a halogen atom, or an amino group; a to 1 and o to s each independently represent a hydrogen atom, an alkyl group having 1 to 20 carbon atoms, a substituted alkyl group having 1 to 20 carbon atoms, an aryl group having 6 to 20 carbon atoms, a substituted aryl group having 6 to 20 carbon atoms, or a halogen atom; the coordinating metal M represents platinum (Pt), iridium (Ir), nickel (Ni), copper (Cu), or gold (Au); and the numbers of repetition, m and n, each independently represent an integer from 0 to 4, while m + n represents an integer of 1 or greater.

Fig.1

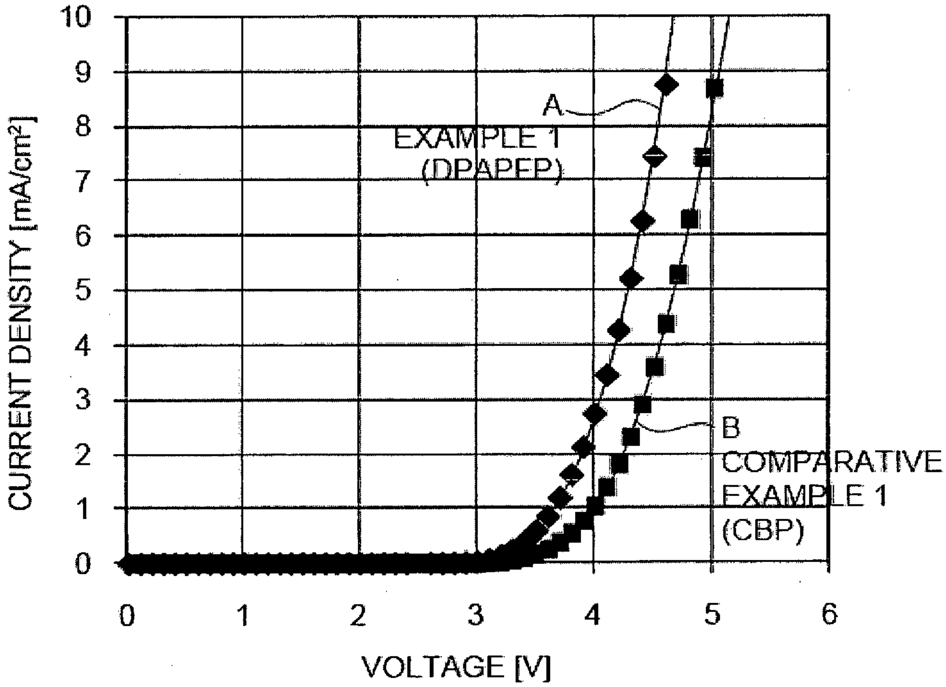
CURRENT DENSITY [mA/cm²]
10
9
8
7
6
5
4
3
2
1
0
0
1
2
3
4
5
6
VOLTAGE [V]
A
EXAMPLE 1
(DPAPFP)
B
COMPARATIVE
EXAMPLE 1
(CBP)

Fig.2(a)

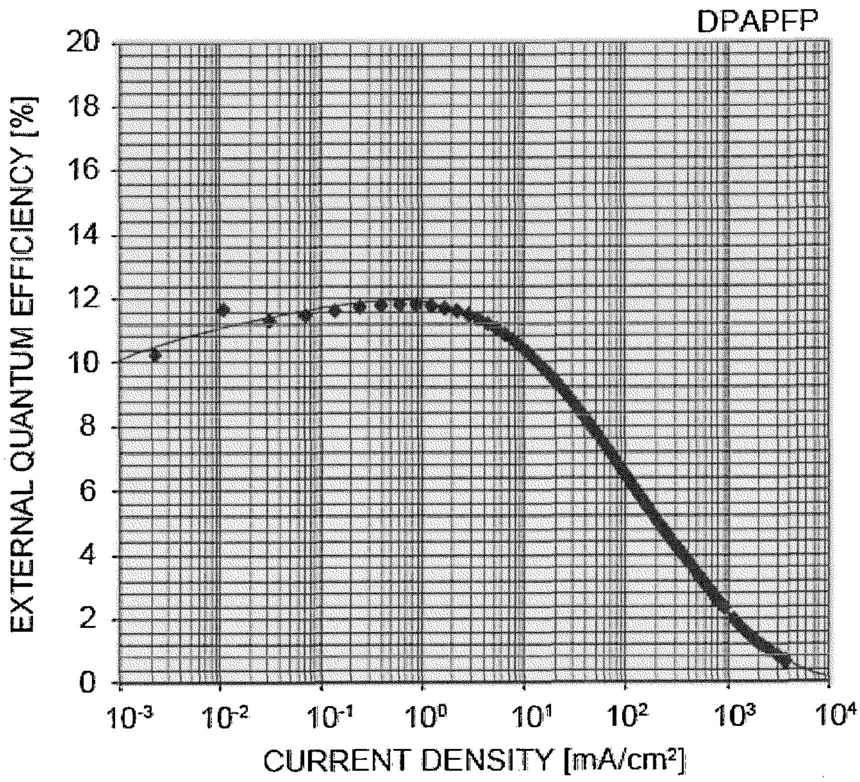
DPAPFP
EXTERNAL QUANTUM EFFICIENCY [%]
20
18
16
14
12
10
8
6
4
2
0
$10^{-3}$ $10^{-2}$ $10^{-1}$ $10^{0}$ $10^{1}$ $10^{2}$ $10^{3}$ $10^{4}$
CURRENT DENSITY [mA/cm$^2$]

Fig.2(b)

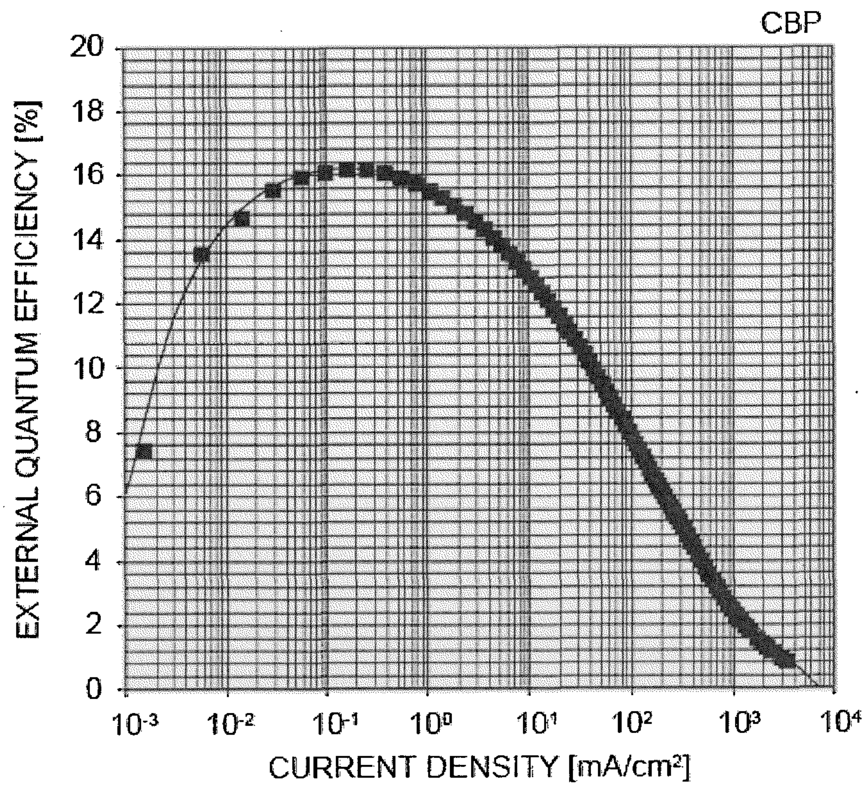
CBP
EXTERNAL QUANTUM EFFICIENCY [%]
20
18
16
14
12
10
8
6
4
2
0
$10^{-3}$ $10^{-2}$ $10^{-1}$ $10^{0}$ $10^{1}$ $10^{2}$ $10^{3}$ $10^{4}$
CURRENT DENSITY [mA/cm$^2$]

Fig.3(a)

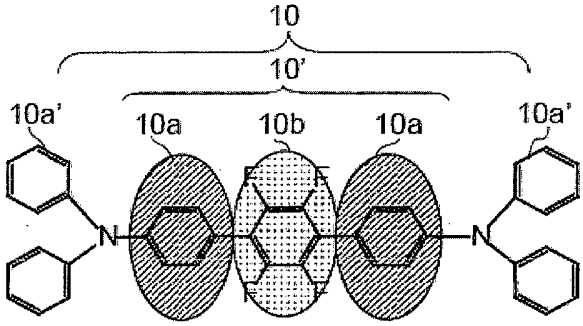


Fig.3(b)

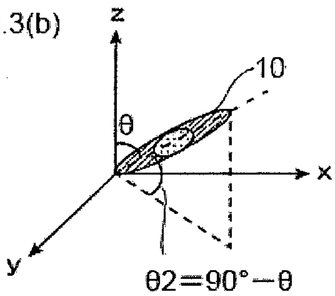


Fig.3(c)

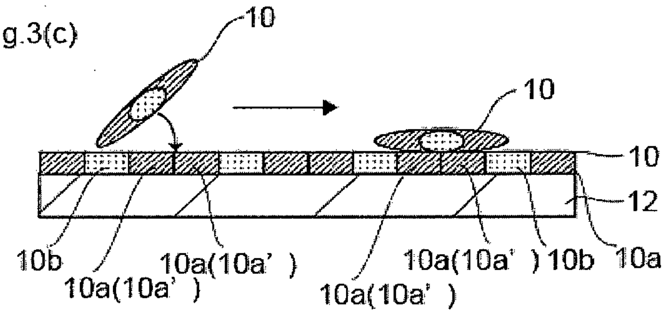


Fig.3(d)

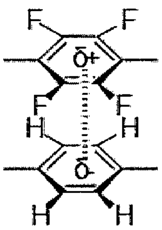

Fig.3(e)

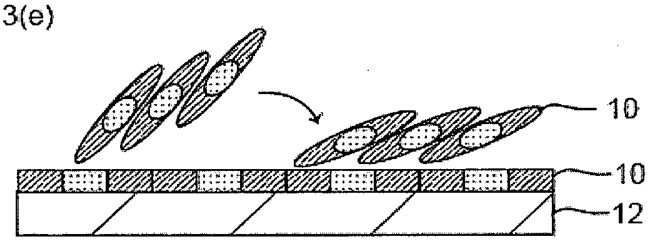


Fig.3(f)

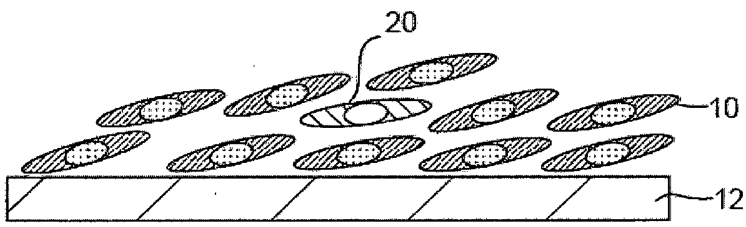

Fig.4(a)

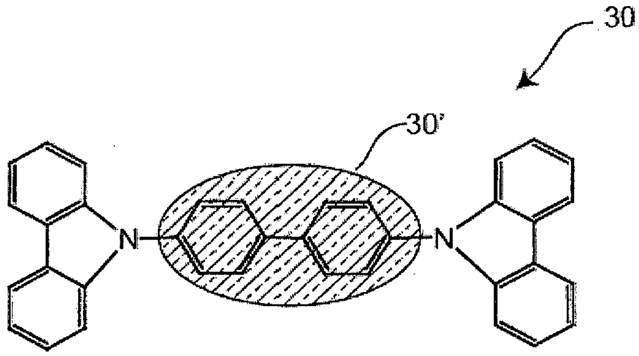


Fig.4(b)

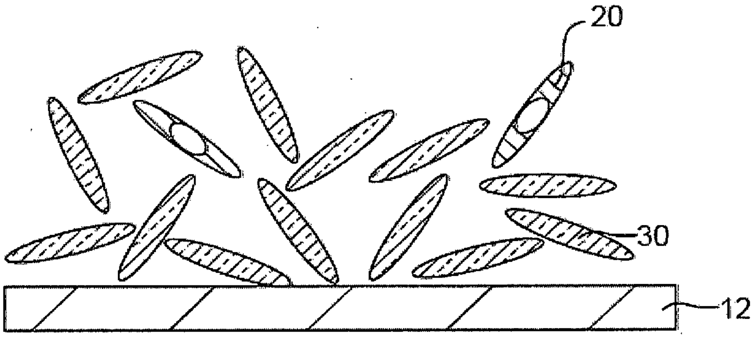


Fig.5

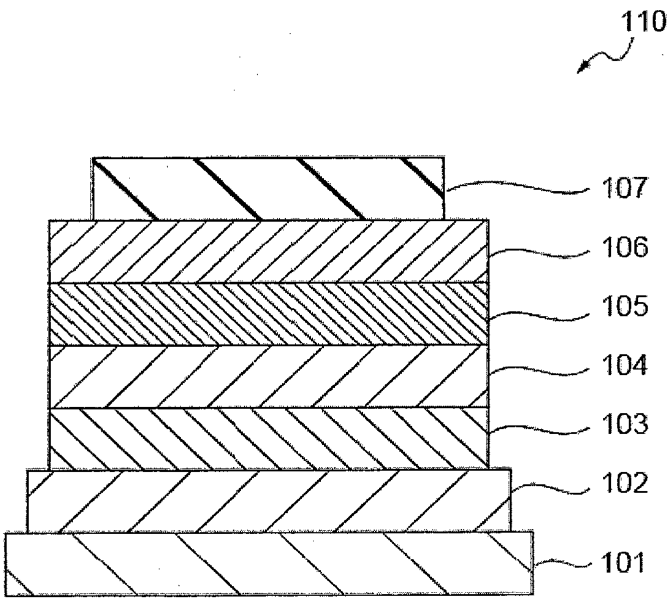

Fig.6

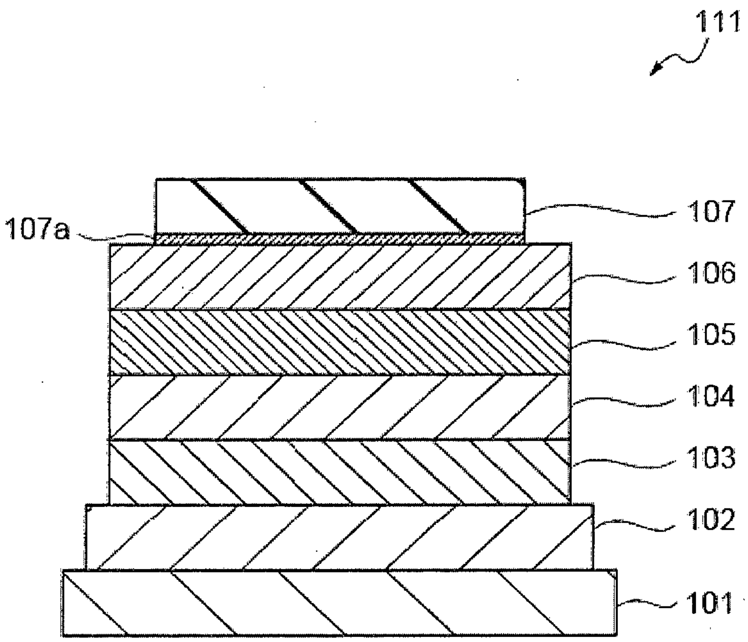
111
107
107a
106
105
104
103
102
101

Fig.7

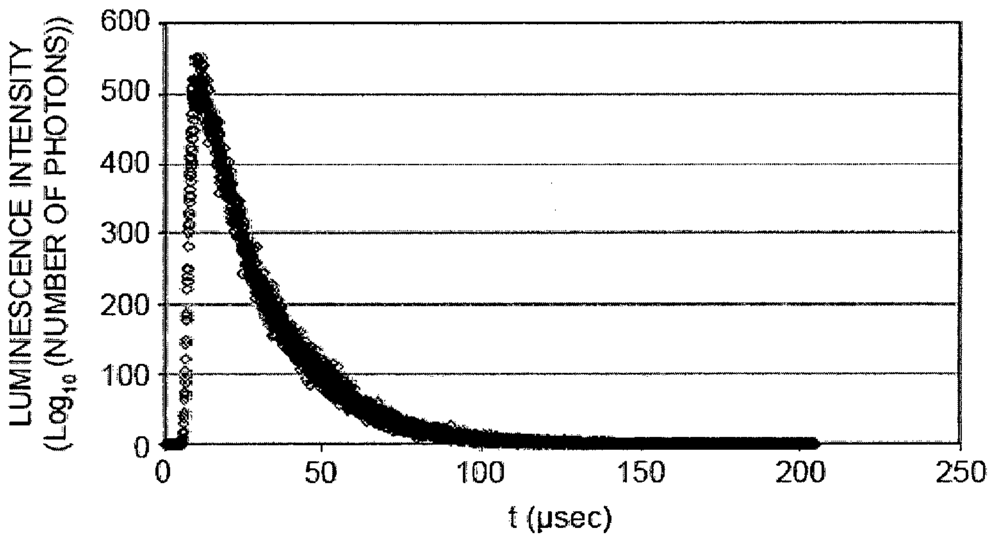
LUMINESCENCE INTENSITY
(Log10 (NUMBER OF PHOTONS))
600
500
400
300
200
100
0
0
50
100
150
200
250
t (μsec)

Fig.8

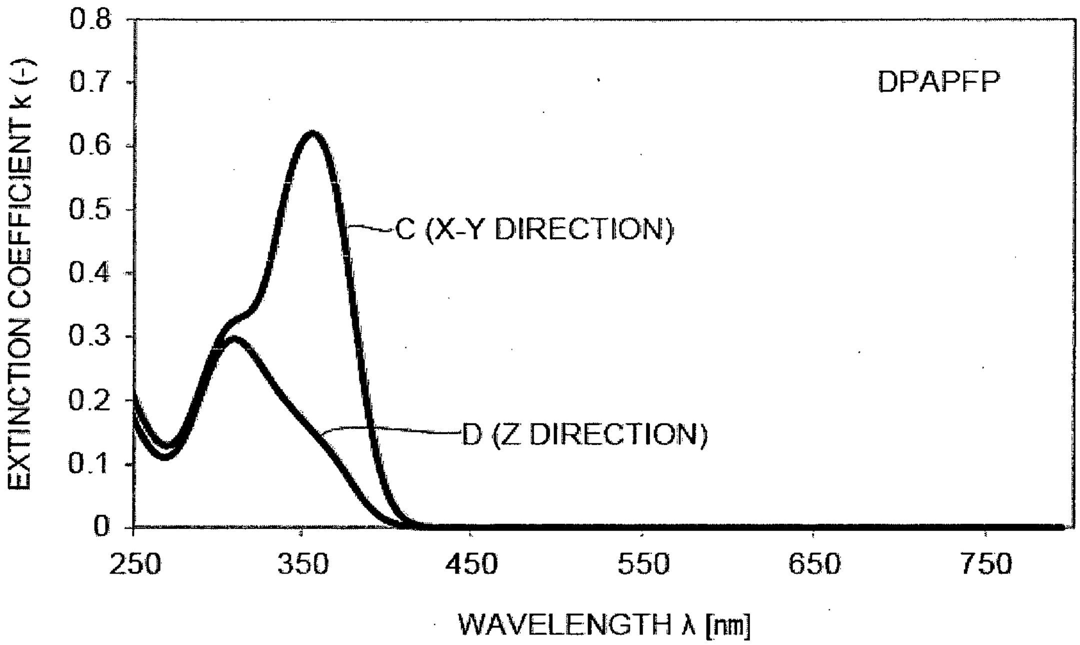

DPAPFP
C (X-Y DIRECTION)
D (Z DIRECTION)
EXTINCTION COEFFICIENT k (-)
0.8
0.7
0.6
0.5
0.4
0.3
0.2
0.1
0
250
350
450
550
650
750
WAVELENGTH λ [nm]

Fig.9

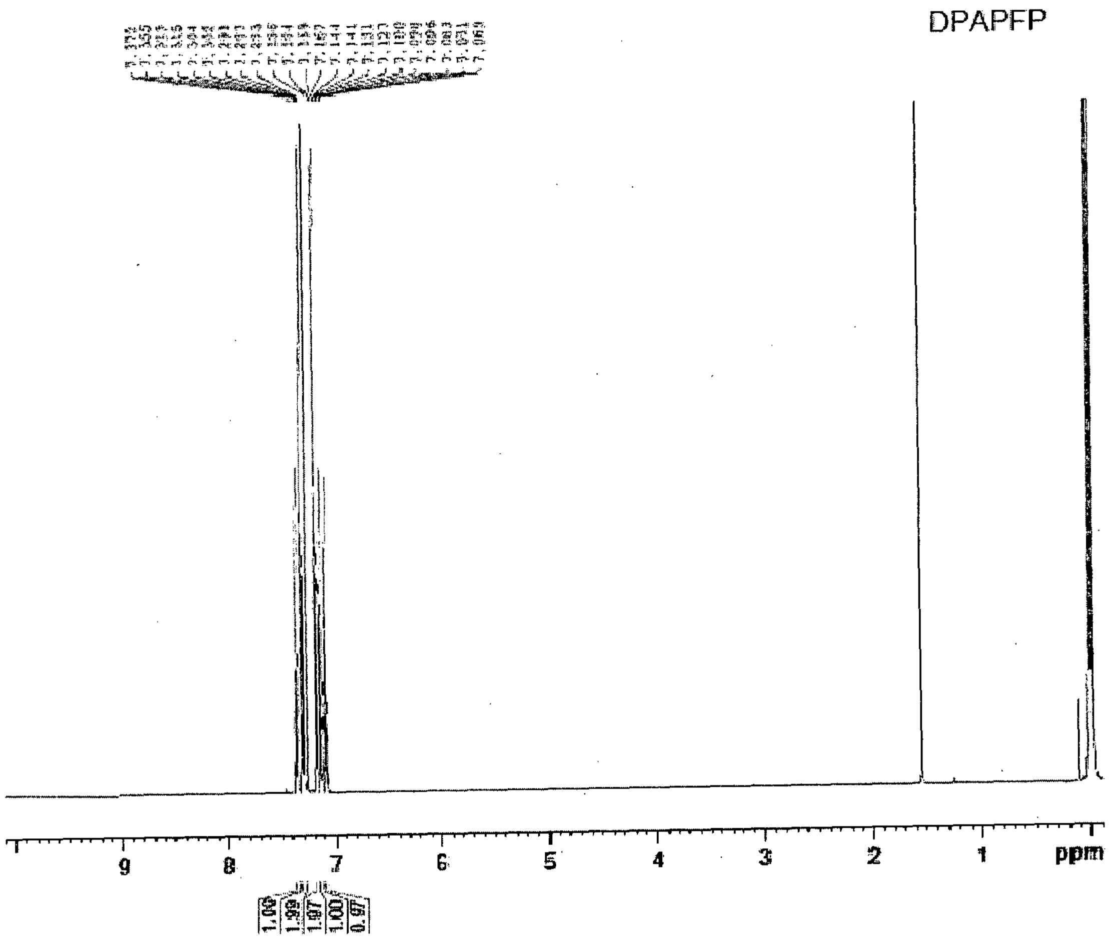

Fig.10

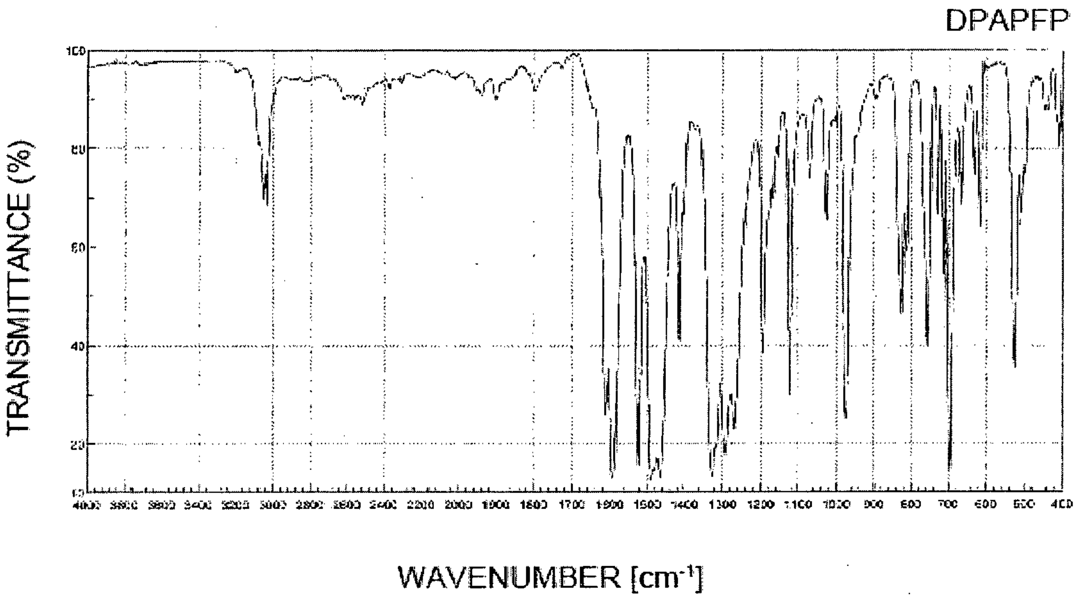

Fig.11

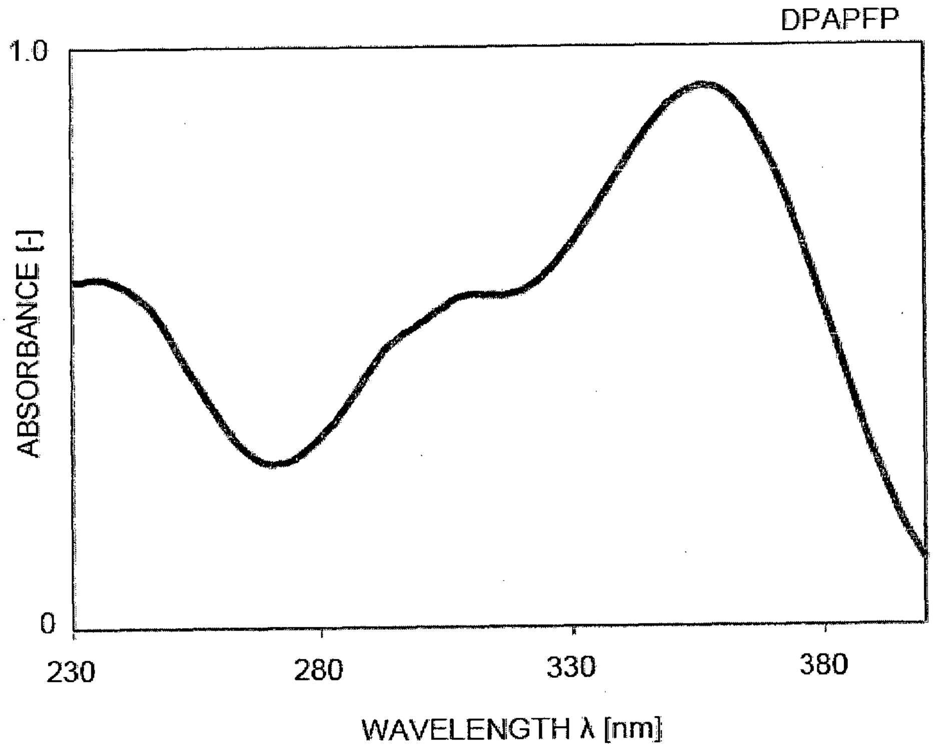

Fig.12

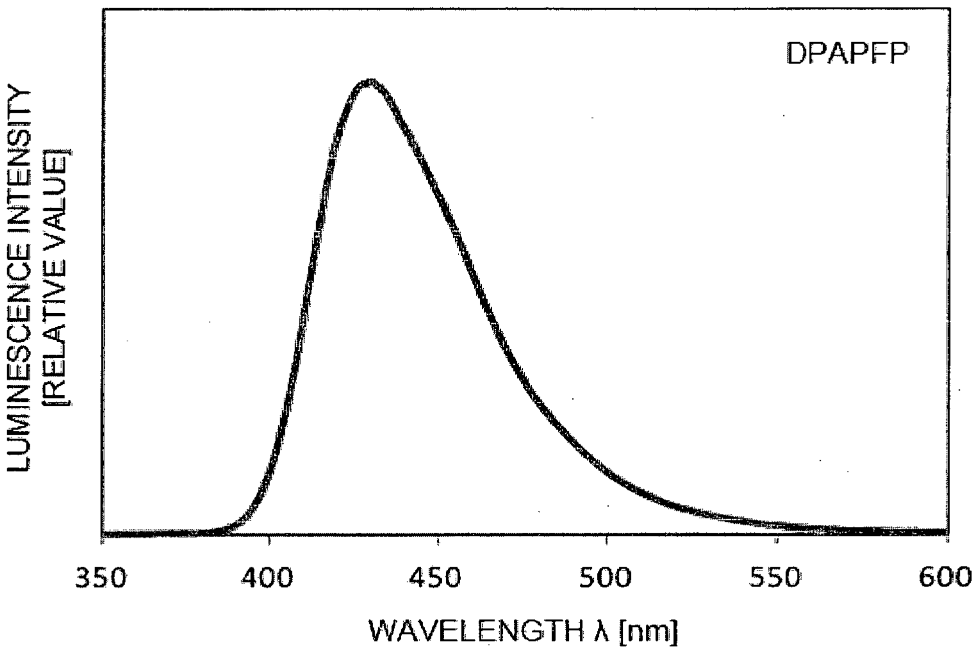
DPAPFP
LUMINESCENCE INTENSITY [RELATIVE VALUE]
350
400
450
500
550
600
WAVELENGTH λ [nm]

Fig.13

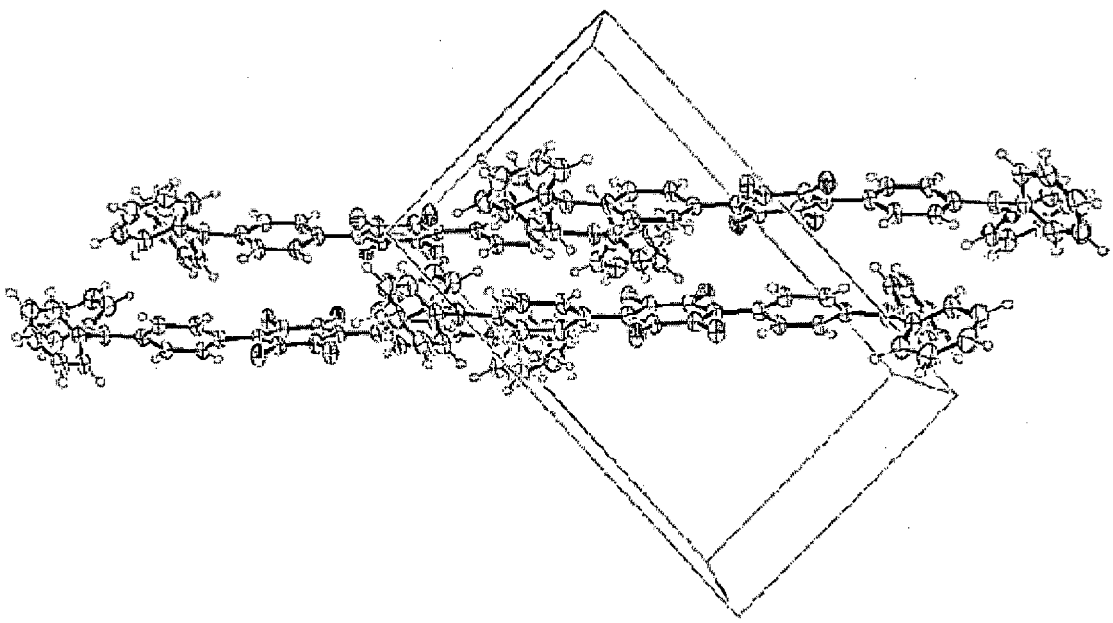

Fig.14(a)

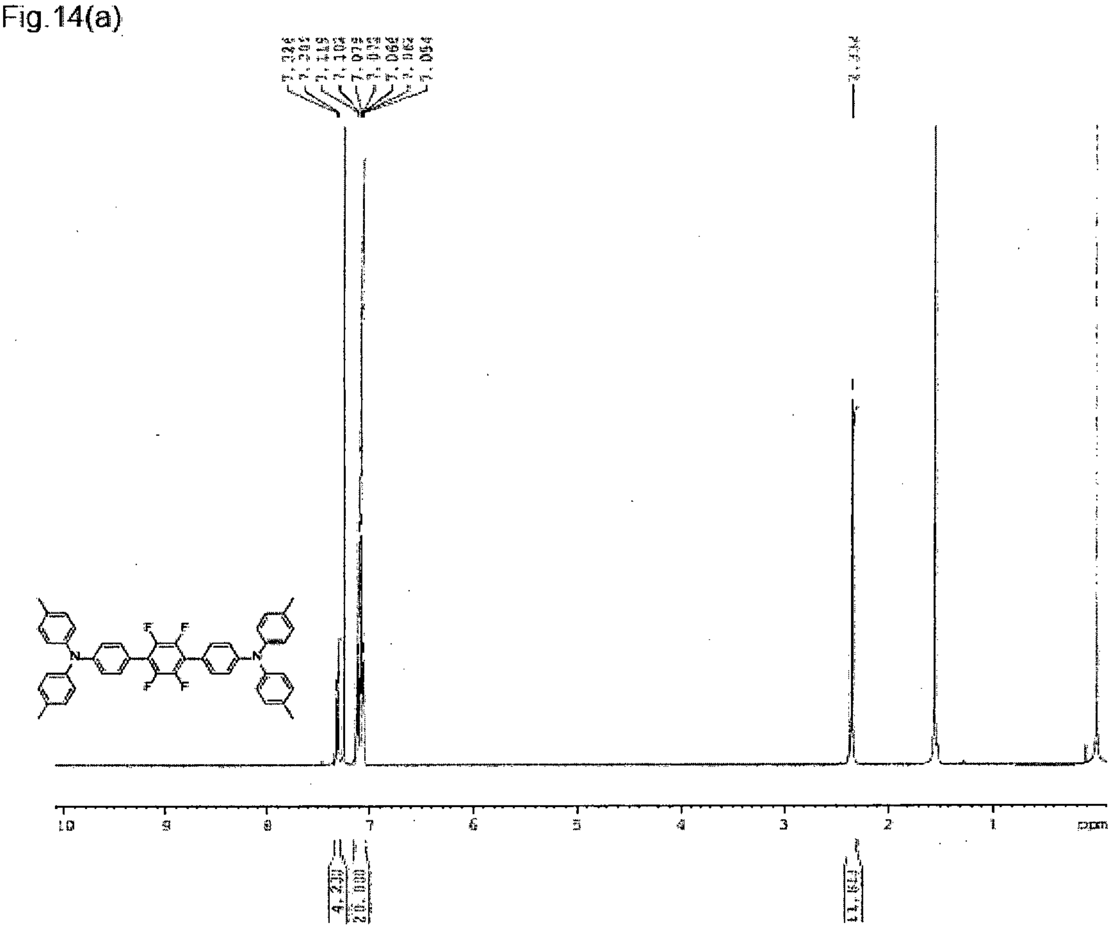


Fig.14(b)

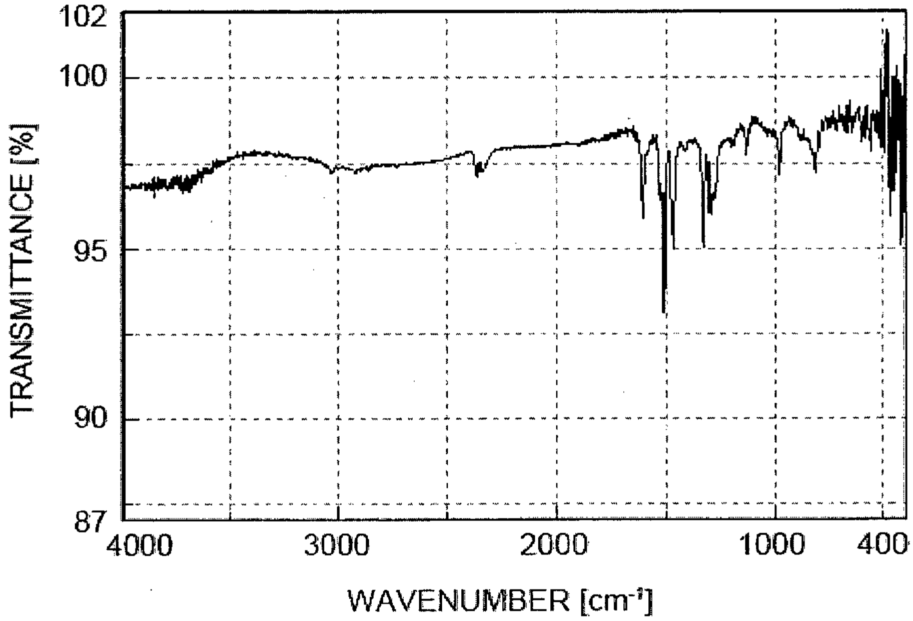

Fig.15(a)

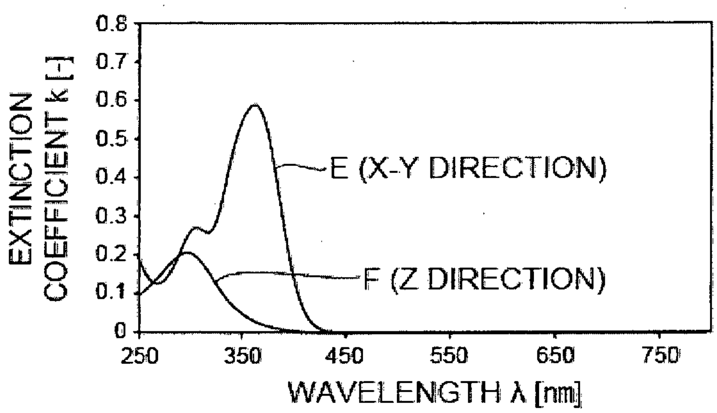


Fig.15(b)

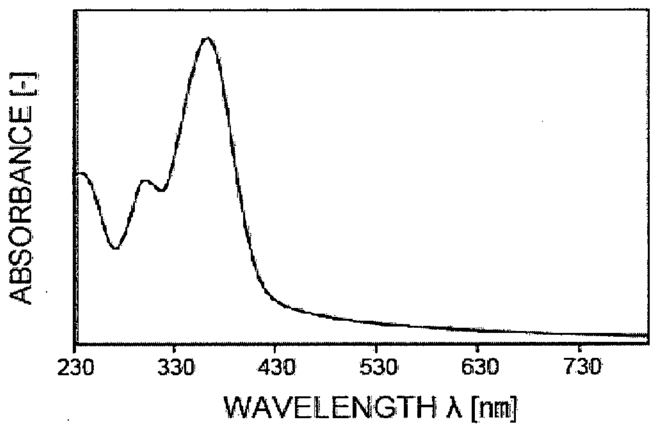


Fig.15(c)

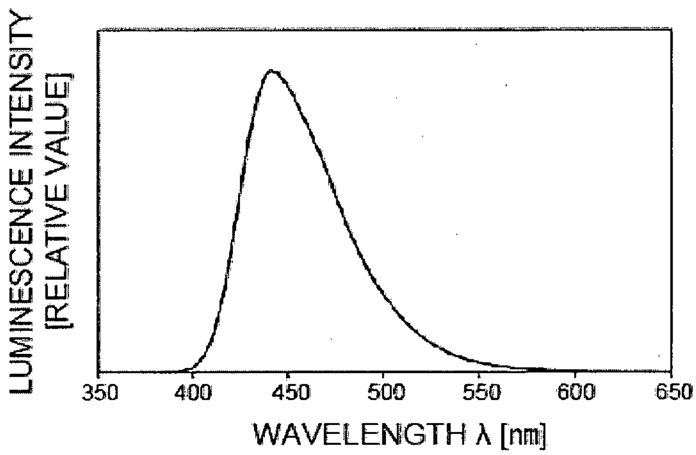

Fig.16(a)

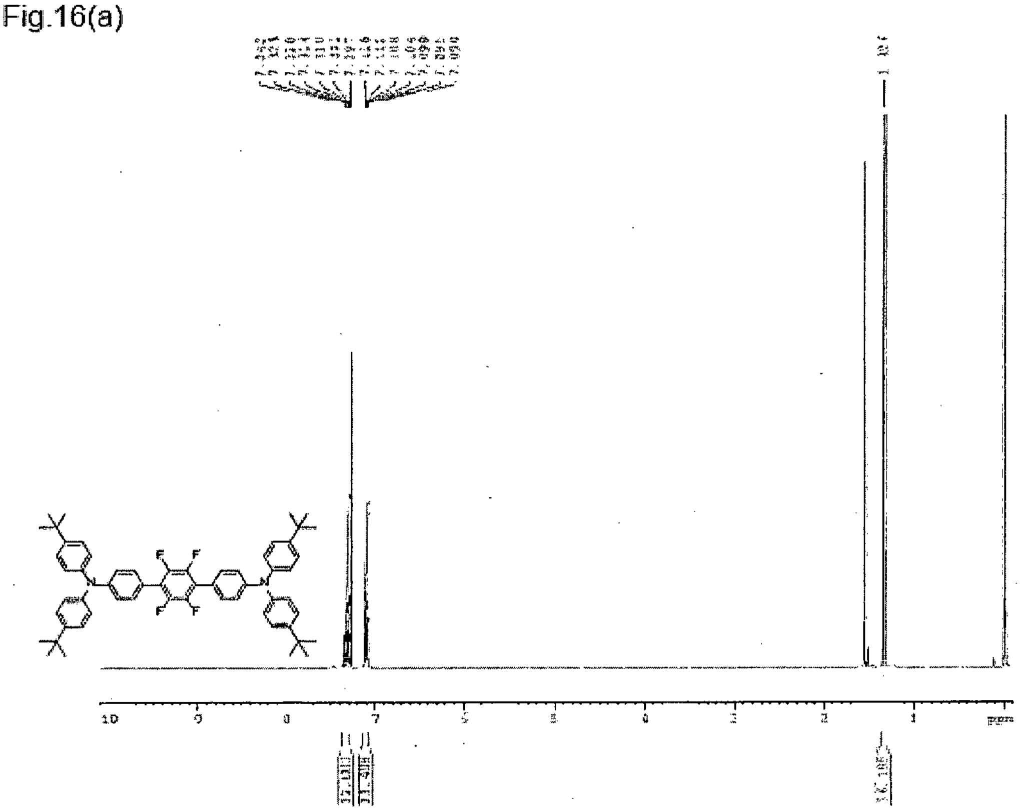

Fig.16(b)

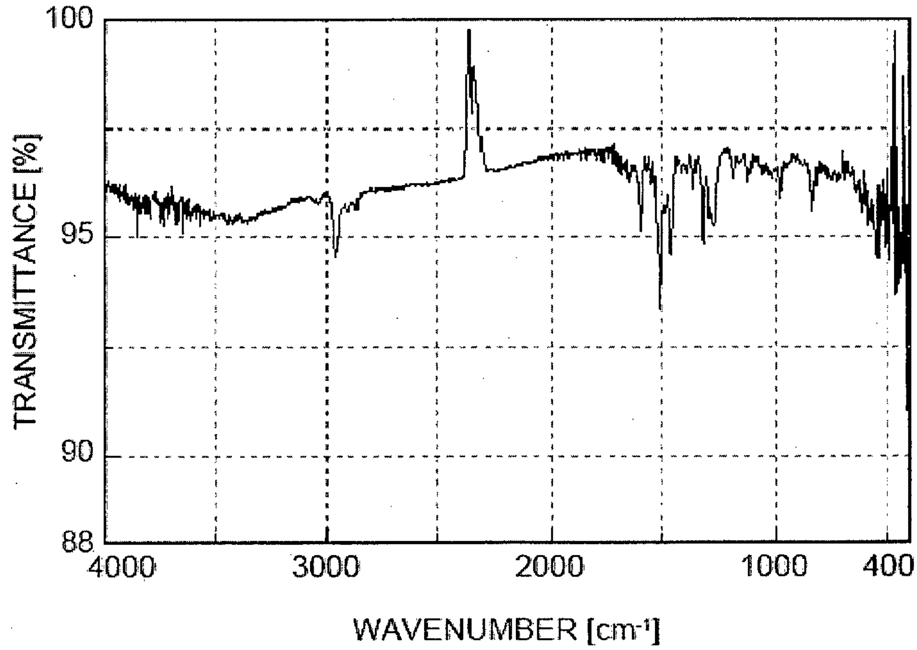

Fig.17(a)

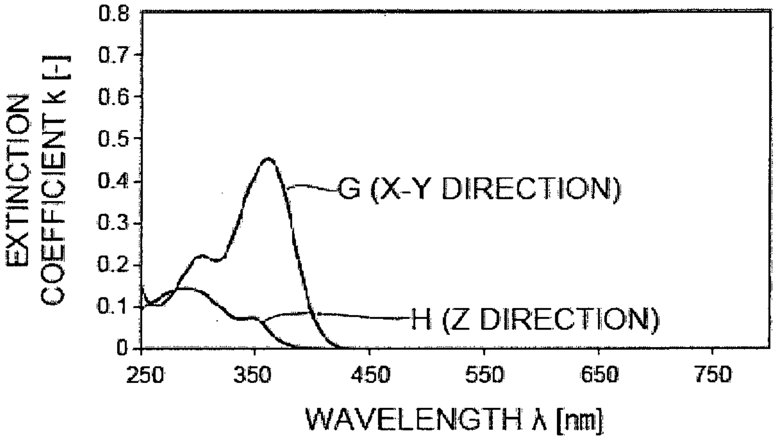


Fig.17(b)

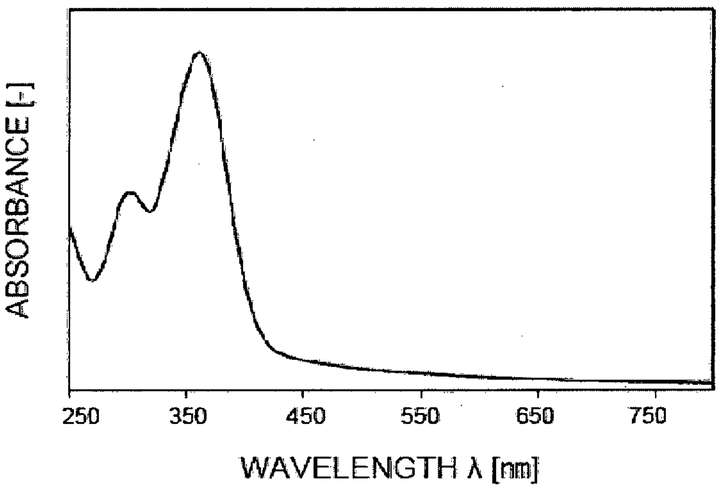


Fig.17(c)

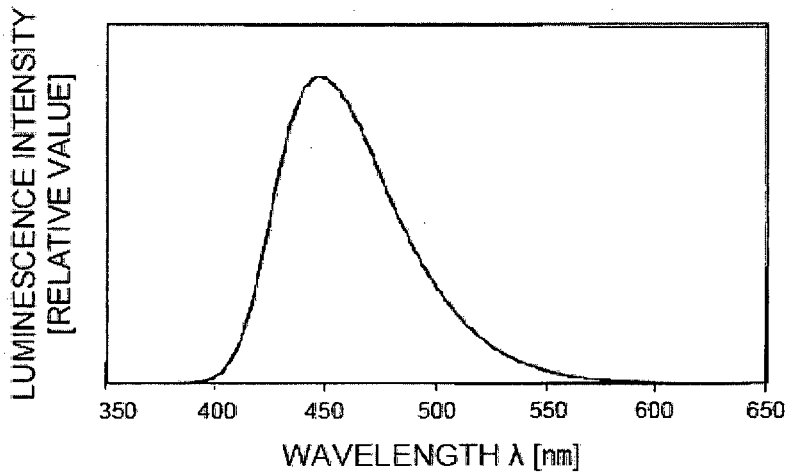

Fig.18(a)

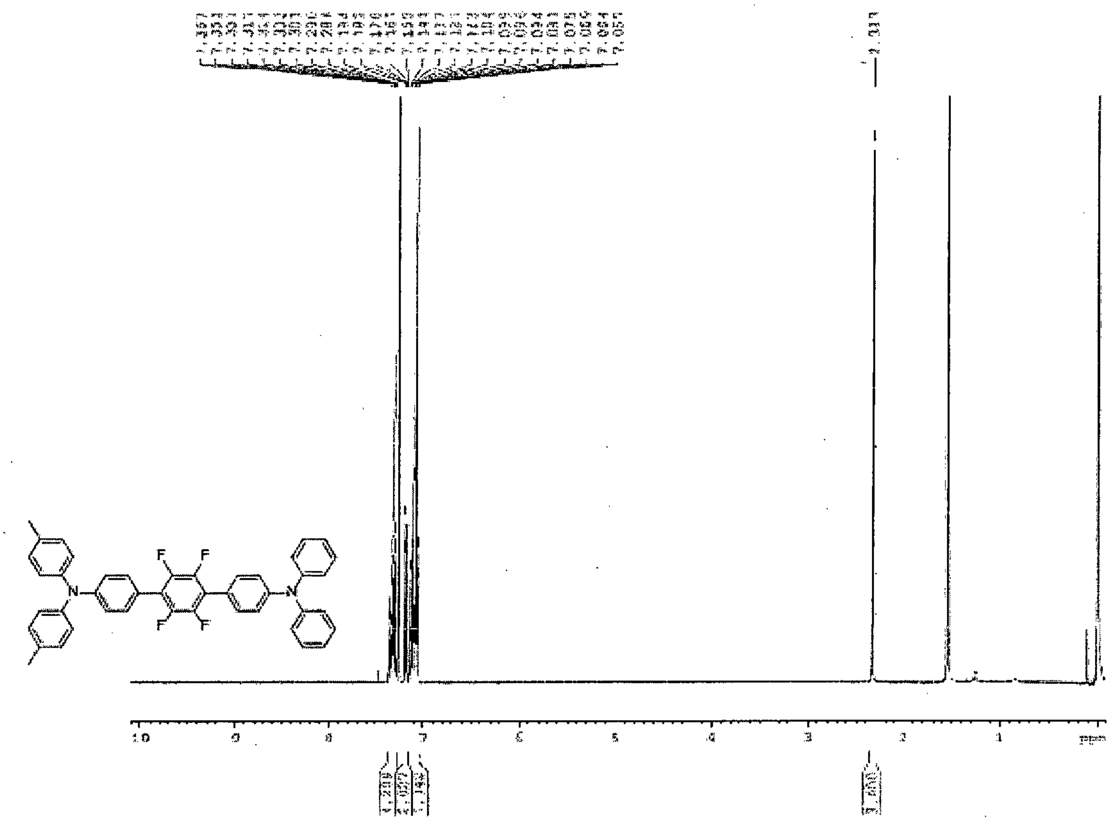

Fig.18(b)

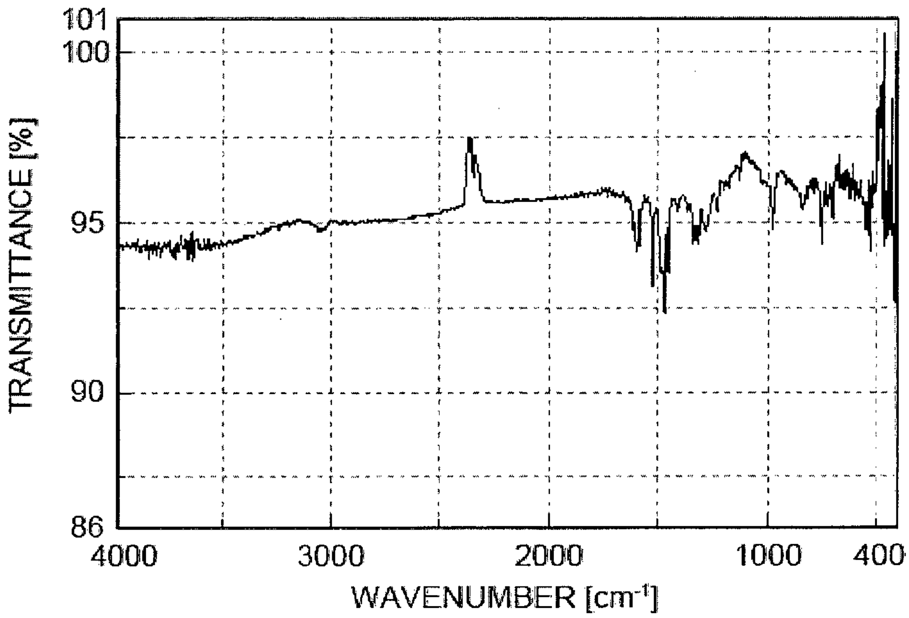

Fig.19(a)

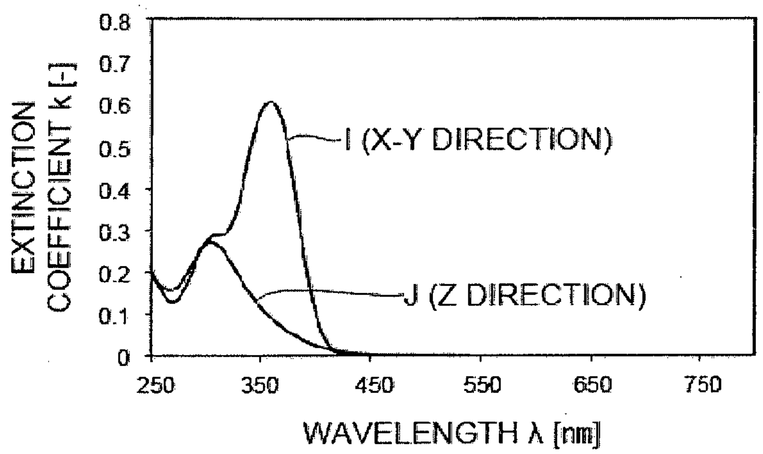


Fig.19(b)

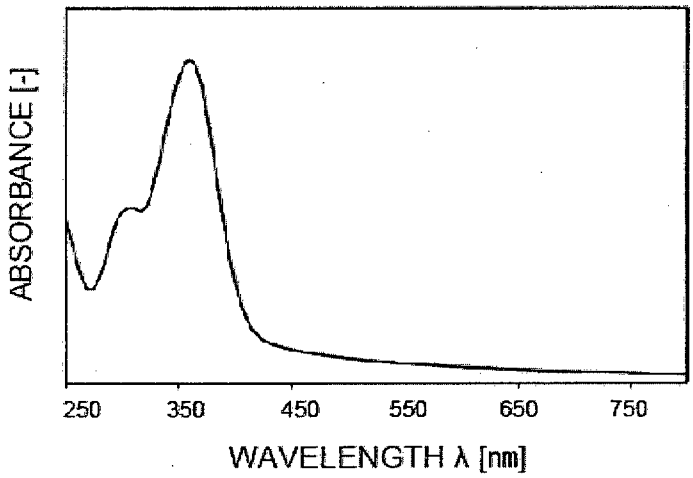


Fig.19(c)

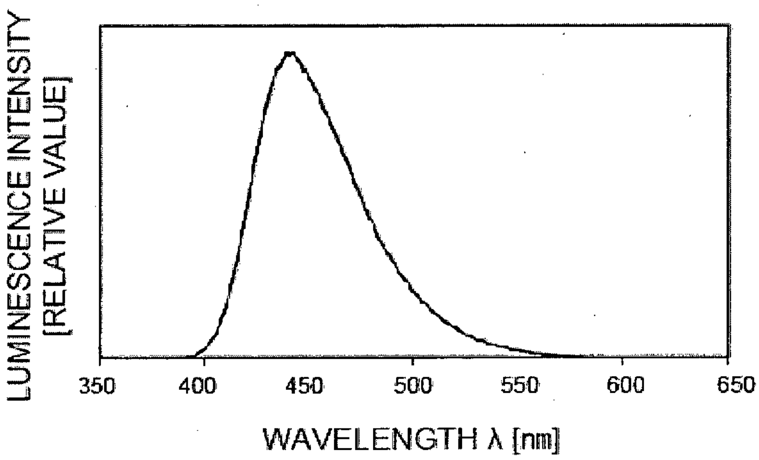

**INTERNATIONAL SEARCH REPORT**

International application No. PCT/JP2013/071132

A. CLASSIFICATION OF SUBJECT MATTER
*C09K11/06*(2006.01)i, *H01L51/50*(2006.01)i, *C07C211/56*(2006.01)n

According to International Patent Classification (IPC) or to both national classification and IPC

B. FIELDS SEARCHED

Minimum documentation searched (classification system followed by classification symbols)
C09K11/06, H01L51/50, C07C211/56

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 4311707 B2 (Canon Inc.), 12 August 2009 (12.08.2009), paragraphs [0012] to [0031]; example 5 & US 2006/0068221 A1 & WO 2004/020548 A1 & AU 2003256084 A | 1-4,9<br>5-8,10,11 |
| Y | JP 10-017531 A (Fuji Photo Film Co., Ltd.), 20 January 1998 (20.01.1998), claim 3; paragraphs [0013] to [0017] (Family: none) | 5-8 |
| Y | WO 2010/112799 A1 (UNIVERSITY OF YORK), 07 October 2010 (07.10.2010), claims; page 1, lines 4 to 8; page 4, lines 8 to 11; scheme 1; experimental (particularly, preparation of complex 22) & GB 903271 D | 10,11 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 October, 2013 (23.10.13) | 05 November, 2013 (05.11.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001313178 A
- JP 2007277170 A
- JP 4311707 B